# EUROPÄISCHE PATENTSCHRIFT

(11) **EP 3 969 435 B1**
(45) Veröffentlichungstag und Bekanntmachung des Hinweises auf die Patenterteilung: **18.09.2024**
(21) Anmeldenummer: 20724537.4
(22) Anmeldetag: 14.05.2020
(51) Int. Cl.: C07C 45/75, C07C 67/08, C07C 251/06, C08G 18/32, C07C 69/14, C08G 18/10, C08G 18/30, C07C 47/19, C08G 18/48, C08G 18/50, C08G 18/76, C09D 175/04, C09J 175/04

(54) **PROZESS FÜR DIE HERSTELLUNG VON 2,2-DIALKYL-3-ACYLOXYPRO-PANALEN**
PROCESS FOR THE PREPARATION OF 2,2-DIALKYL-3-ACYLOXY PROPANALS
PROCÉDÉ DE PRODUCTION DE 2,2-DIALKYL-3-ACYLOXYPROPANALES

(30) Priorität: 17.05.2019 EP 19175218
(43) Veröffentlichungstag der Anmeldung: 23.03.2022
(73) Patentinhaber: Sika Technology AG, 6340 Baar (CH)
(72) Erfinder: BURCKHARDT, Urs, 8049 Zürich (CH); KRAMER, Andreas, 8008 Zürich (CH); GEYER, Michael, 8200 Schaffhausen (CH); BARRATT, John, Manchester M251EG (GB)
(74) Vertreter: Sika Patent Attorneys
(86) Internationale Anmeldenummer: PCT/EP2020/063538
(87) Internationale Veröffentlichungsnummer: WO 2020/234128

(56) Entgegenhaltungen:
- EP-A1- 1 975 190
- WO-A1-2015/135914
- US-A- 3 808 280
- US-A1- 2007 282 135
- US-A1- 2015 239 809
- HOSOKAWA S ET AL: "Total synthesis of madindoline A", TETRAHEDRON LETTERS, ELSEVIER, AMSTERDAM, NL, vol. 41, no. 33, 12 August 2000 (2000-08-12), pages 6435 - 6439, XP004215082, ISSN: 0040-4039, DOI: 10.1016/S0040-4039(00)00939-4
- DATABASE WPI 1 January 2017 Derwent World Patents Index; AN 2015-641959, XP002795062, CHO J.E. ET AL.: "Preparation aqueous solution base catalyst react isobutyraldehyde aldol presence"

## Beschreibung

### Technisches Gebiet

Herstellung von Aldolester-Aldehyden und -Aldiminen und feuchtigkeitshärtende Polyurethan-Zusammensetzungen enthaltend diese, insbesondere für die Anwendung als Klebstoff, Dichtstoff oder Beschichtung.

### Stand der Technik

2,2-Dialkyl-3-acyloxypropanale stellen Carbonsäureester von Aldolen aus der gekreuzten Aldolreaktion sekundärer aliphatischer Aldehyde mit Formaldehyd dar. Sie sind vielseitige Ausgangsstoffe für die Herstellung von beispielsweise Duftstoffen, Farbstoffen und Polymeren. Kommerziell besonders interessant ist ihre Anwendung als Blockierungsmittel für primäre Polyamine. Die damit erhaltenen Aldolester-Aldimine sind besonders geeignet als latente Härter für Isocyanatgruppen-haltige Polymere. Sie ermöglichen Polyurethan-Zusammensetzungen mit guter Lagerstabilität, welche bei Kontakt mit Feuchtigkeit schnell und prozesssicher aushärten und dabei mechanisch hochwertige und beständige Elastomere bilden, wie beispielsweise beschrieben in EP 1,527,115 oder WO 2016/005457. Die Herstellung von 2,2-Dialkyl-3-acyloxypropanalen ist in der Literatur vielfach beschrieben worden. In den bekannten Herstellverfahren wird das Aldol 2,2-Dialkyl-3-hydroxypropanal entweder als solches eingesetzt oder in situ aus den Ausgangsaldehyden erzeugt und mit einer Carbonsäure, seltener auch einem Anhydrid oder Enolester davon, zum Aldolester 2,2-Dialkyl-3-acyloxypropanal verestert. Die Veresterung und gleichzeitig auch die Aldolisierung wird typischerweise in Gegenwart von sauren Katalysatoren wie Schwefelsäure oder p-Toluolsulfonsäure durchgeführt und der Aldolester anschliessend isoliert und aufgereinigt, insbesondere durch Destillation, wie beispielsweise beschrieben in US 3,251,876, US 3,374,267 oder US 3,720,705.

Die Nachteile der beschriebenen Herstellverfahren liegen darin, dass sie in der Praxis eine relativ niedrige Produktausbeute liefern. Das so erhaltene Reaktionsprodukt ist typischerweise stark dunkel gefärbt, riecht aufgrund von geruchsintensiven Nebenprodukten stechend und muss zur weiteren Verwendung aufgereinigt werden. Zudem zeigt die Erfahrung, dass der Herstellprozess, insbesondere für kurzkettige Aldolester, unter saurer Katalyse thermische Prozessrisiken birgt, die in einer grossen Produktionsanlage keinen sicheren Betrieb ermöglichen. Dies gilt sowohl für die Umsetzung selbst, insbesondere wenn ohne ein die Reaktionstemperatur begrenzendes Löse- bzw. Schleppmittel gearbeitet wird, wie auch nach der Umsetzung bei der Aufreinigung des Reaktionsprodukts, insbesondere durch Destillation über Kopf. So kommt es bereits bei einer Temperatur im Bereich von 150 °C zu stark exothermen Zersetzungsreaktionen, die auch durch eine nachträgliche Neutralisation des Säurekatalysators nicht ausreichend unterdrückt werden. Ausserdem erfordern die stark sauren Bedingungen, dass die Herstellung in korrosionsfesten Anlagen durchgeführt wird. Das in US 4,017,537 beschriebene Herstellverfahren ohne Katalysator unter neutralen Bedingungen oder das in DE 19,506,728 beschriebene Herstellverfahren mit Pyridin als Katalysator verursachen zwar keine thermischen Prozessrisiken und keine Korrosionsprobleme. Sie sind aber ebenfalls unbefriedigend, da die Umsetzung sehr lange dauert und relativ niedrige Ausbeuten liefert. S. Hosokawa et al., Tetrahedron Lett., 14, 6435-6439 (2000) betrifft ein zweistufiges Herstellungsverfahren eines Aldolesters über eine Chlorid-Zwischenverbindung.

### Darstellung der Erfindung

Aufgabe der vorliegenden Erfindung ist es daher, ein Verfahren zur Herstellung von 2,2-Dialkyl-3-acyloxypropanalen bereitzustellen, das eine hohe Produktausbeute liefert und bei guter Raum-Zeit-Ausbeute ohne thermische Prozessrisiken durchführbar ist.

Diese Aufgabe wird mit dem Verfahren wie in Anspruch 1 beschrieben gelöst. Dabei wird ein Carbonsäureanhydrid mit einem Aldol unter Erwärmen in Gegenwart eines basischen Katalysators mit einem pKa-Wert der konjugierten Säure von mindestens 8 umgesetzt. Ein solches Verfahren mit einem basischen Katalysator ist bisher nicht beschrieben. Überraschenderweise wurde gefunden, dass das erfindungsgemässe Verfahren eine schnelle Umsetzung ohne thermische Prozessrisiken bei hoher Ausbeute ermöglicht, wobei auf ein Löse- oder Schleppmittel verzichtet werden kann. Das erhaltende Reaktionsprodukt ist überraschend hellfarbig und geruchsarm und kann somit auch ohne aufwendige Aufreinigung, insbesondere ohne Destillation über Kopf, verwendet werden, insbesondere als Blockierungsmittel für primäre Amine. Weil keine Korrosionswirkung auf Metalle besteht, kann das erfindungemässe Verfahren in preisgünstigen Standardreaktoren aus rostfreiem Stahl durchgeführt werden. Besonders überraschend beim erfindungsgemässen Verfahren ist der Umstand, dass das Reaktionsprodukt auch im Fall von kurzkettigen Aldolestern, insbesondere 2,2-Dialkyl-3-acetyloxypropanalen, beim Erhitzen bis weit über 200°C stabil ist, während bei entsprechenden Reaktionsprodukten aus säurekatalysierten Prozessen beim Erhitzen ab 150°C eine hohe Exothermie beobachtet wird, was ein erhebliches thermisches Prozessrisiko bedeutet.

Das erfindungsgemässe Verfahren ermöglicht ein hellfarbiges und geruchsarmes Reaktionsprodukt mit hohem Gehalt an 2,2-Dialkyl-3-acyloxypropanal, das ohne aufwendige Aufreinigungsschritte, insbesondere ohne Destillation des 2,2-Dialkyl-3-acyloxypropanals über Kopf, als Blockierungsmittel für primäre Amine eingesetzt werden kann. Die daraus erhaltenen blockierten Amine bzw. latenten Härter sind geruchsarm, zusammen mit Isocyanatgruppen-haltigen Polymeren überraschend lagerstabil, härten bei Kontakt mit Feuchtigkeit schnell und prozesssicher aus und bilden dabei mechanisch hochwertige und beständige Elastomere. Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Gegenstand der Erfindung ist ein Verfahren zur Herstellung eines Aldolesters der Formel (I), 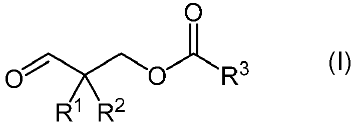 wobei R¹ und R² für gleiche oder verschiedene Alkyl-Reste mit 1 bis 4 C-Atomen oder zusammen für einen Alkylen-Rest mit 4 bis 6 C-Atomen stehen und R³ für einen gegebenenfalls halogenierten Kohlenwasserstoff-Rest mit 1 bis 17 C-Atomen steht,
dadurch gekennzeichnet, dass mindestens ein Carbonsäureanhydrid der Formel (II) 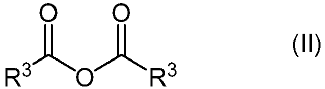 mit mindestens einem Aldol der Formel (III), 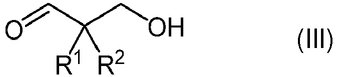 gegebenenfalls in Form eines Oligomers davon, unter Erwärmen in Gegenwart eines basischen Katalysators mit einem pKa-Wert der konjugierten Säure von mindestens 8 umgesetzt wird.

Als "aliphatisch" wird eine Aldehyd- oder eine Isocyanatgruppe bezeichnet, welche direkt an ein aliphatisches oder cycloaliphatisches C-Atom gebunden ist.

Als "aromatisch" wird eine Aldehyd- oder eine Isocyanatgruppe bezeichnet, welche direkt an ein aromatisches C-Atom gebunden ist.

Als "primäre Aminogruppe" wird eine Aminogruppe bezeichnet, die an einen einzigen organischen Rest gebunden ist und zwei Wasserstoffatome trägt; als "sekundäre Aminogruppe" wird eine Aminogruppe bezeichnet, die an zwei organische Reste, welche auch gemeinsam Teil eines Rings sein können, gebunden ist und ein Wasserstoffatom trägt; und als "tertiäre Aminogruppe" wird eine Aminogruppe bezeichnet, die an drei organische Reste, welche auch zu zweit oder zu dritt Teil eines oder mehrerer Ringe sein können, gebunden ist und kein Wasserstoffatom trägt.

Mit "Poly" beginnende Substanznamen wie Polyamin, Polyol oder Polyisocyanat bezeichnen Substanzen, die formal zwei oder mehr der in ihrem Namen vorkommenden funktionellen Gruppen pro Molekül enthalten.

Als "Molekulargewicht" wird die molare Masse (in Gramm pro Mol) eines Moleküls oder eines Molekül-Rests bezeichnet. Als "mittleres Molekulargewicht" wird das Zahlenmittel des Molekulargewichts (Mₙ) einer polydispersen Mischung von oligomeren oder polymeren Molekülen oder Molekül-Resten bezeichnet. Es wird mittels Gelpermeationschromatographie (GPC) gegen Polystyrol als Standard bestimmt.

Gewichtsprozente (Gewichts-%), bezeichnen Massenanteile eines Bestandteils einer Zusammensetzung bezogen auf die gesamte Zusammensetzung, falls nichts anderes angeben ist. Die Begriffe "Masse" und "Gewicht" werden im vorliegenden Dokument synonym benutzt.

Als "NCO-Gehalt" wird der Gehalt an Isocyanatgruppen in Gewichts-% bezeichnet. Als "lagerstabil" oder "lagerfähig" wird eine Substanz oder eine Zusammensetzung bezeichnet, wenn sie bei Raumtemperatur in einem geeigneten Gebinde während längerer Zeit, typischerweise während mindestens 3 Monaten bis zu 6 Monaten und mehr, aufbewahrt werden kann, ohne dass sie sich in ihren Anwendungs-oder Gebrauchseigenschaften durch die Lagerung in einem für ihren Gebrauch relevanten Ausmass verändert.

Als "Raumtemperatur" wird eine Temperatur von 23°C bezeichnet.

Alle im Dokument erwähnten Industriestandards und Normen beziehen sich auf die zum Zeitpunkt der Einreichung der Erstanmeldung gültigen Fassungen.

Bevorzugt stehen R¹ für Methyl oder Ethyl, insbesondere für Methyl, und R² für Methyl, Ethyl, n-Propyl oder n-Butyl.

Besonders bevorzugt stehen R¹ und R² jeweils für Methyl.

Bevorzugt steht R³ für einen gegebenenfalls chlorierten Kohlenwasserstoff-Rest mit 1 bis 11 C-Atomen.

Besonders bevorzugt steht R³ für einen Alkyl-Rest mit 1 bis 7 C-Atomen oder für Phenyl.

Am meisten bevorzugt steht R³ für Methyl.

Die bevorzugten Reste R¹, R² und R³ sind besonders einfach zugänglich und ermöglichen Aldolester der Formel (I), welche besonders geeignet sind als Blockierungsmittel für primäre Amine.

Im Fall von kleinen Resten R³, insbesondere Methyl, ist das erfindungsgemässe Verfahren besonders vorteilhaft, da bei den bekannten säurekatalysierten Verfahren nach dem Stand der Technik stark gefärbte, geruchsintensive und thermisch unstabile Reaktionsprodukte mit hohem Prozessrisiko entstehen. Blockierte Amine auf der Basis von Aldolestern der Formel (I) mit kleinen Resten R³, insbesondere Methyl, sind besonders geeignet für feuchtigkeitshärtende Polyurethan-Zusammensetzungen, welche eine besonders niedrige Viskosität und/oder eine besonders hohe Härte aufweisen sollen, beispielsweise für Beschichtungen.

Der basische Katalysator weist bevorzugt einen pKa-Wert der konjugierten Säure von mindestens 9, insbesondere mindestens 10, auf. Damit wird eine besonders schnelle Umsetzung erreicht.

Bevorzugt ist der basische Katalysator ein tertiäres Amin oder ein Amidin. Besonders bevorzugt ist der basische Katalysator ausgewählt aus der Gruppe bestehend aus Trimethylamin, Dimethylethylamin, Methyldiethylamin, Triethylamin, Diisopropylethylamin, N-Methylpyrrolidin, N-Methylpiperidin, 1,5-Diazabicyclo[4.3.0]non-5-en (DBN) und 1,8-Diazabicyclo[5.4.0]undec-7-en (DBU). Diese Verbindungen sind einfach verfügbar und zeigen eine gute katalytische Wirkung im erfindungsgemässen Verfahren.

Am meisten bevorzugt ist Triethylamin. Dieses ermöglicht eine besonders schnelle Umsetzung, ist kostengünstig und leichtflüchtig und somit aus der Reaktionsmischung mittels Destillation gut entfernbar. Ausserdem eignet es sich auch ausgezeichnet als Katalysator für die vorgängige Herstellung des Aldols der Formel (III).

Der basische Katalysator wird bevorzugt in einer Menge im Bereich von 0.01 bis 10 Gewichts-%, insbesondere 0.05 bis 5 Gewichts-%, bezogen auf die gesamte Reaktionsmischung, eingesetzt.

Das als Katalysator am meisten bevorzugte Triethylamin wird bevorzugt in einer Menge im Bereich von 0.1 bis 10 Gewichts-%, insbesondere 0.5 bis 5 Gewichts-%, bezogen auf die gesamte Reaktionsmischung, eingesetzt.

Bevorzugt wird das Verfahren bei einer Temperatur im Bereich von 80 bis 150°C, insbesondere 100 bis 130°C, durchgeführt.

Bevorzugt wird das Carbonsäureanhydrid der Formel (II) im stöchiometrischen Überschuss in Bezug auf das Aldol der Formel (III) eingesetzt.

Bevorzugt wird das Aldol der Formel (III) vorgelegt und in Gegenwart des basischen Katalysators mit dem Carbonsäureanhydrid der Formel (II) versetzt.

Die aus dem Carbonsäureanhydrid freigesetzte Carbonsäure, nicht umgesetztes Carbonsäureanhydrid, der basische Katalysator und gegebenenfalls vorhandene leichtflüchtige Nebenprodukte und Lösemittel werden bevorzugt während oder nach der Umsetzung zu einem Grossteil oder vollständig aus der Reaktionsmischung entfernt, insbesondere mittels Destillation unter Vakuum.

Gegebenenfalls kann ein Löse- oder Schleppmittel eingesetzt werden, insbesondere Cyclohexan oder Toluol oder ein Kohlenwasserstoffgemisch wie Petroleumbenzin oder hydrotreated naphtha light, insbesondere mit einem Siedebereich von 75 bis 95°C oder 80 bis 100°C.

Bevorzugt wird das Verfahren ohne die Verwendung eines organischen Löse-oder Schleppmittels durchgeführt.

Das Carbonsäureanhydrid der Formel (II) ist bevorzugt ausgewählt aus der Gruppe bestehend aus Essigsäureanhydrid, Propionsäureanhydrid, Buttersäureanhydrid, Isobuttersäureanhydrid, Hexansäureanhydrid, 2-Ethylhexansäureanhydrid, Laurinsäureanhydrid, Benzoesäureanhydrid, Chloressigsäureanhydrid, Dichloressigsäureanhydrid und Trichloressigsäureanhydrid.

Davon bevorzugt sind Essigsäureanhydrid, Propionsäureanhydrid, Hexansäureanhydrid, 2-Ethylhexansäureanhydrid oder Benzoesäureanhydrid.

Am meisten bevorzugt ist Essigsäureanhydrid.

Das Aldol der Formel (III) wird gegebenenfalls in Form eines Oligomers eingesetzt, insbesondere in Form eines Dimers der Formel (III a).

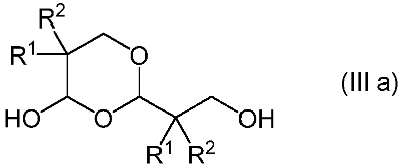

Das Aldol der Formel (III) wird als Bestandteil einer Reaktionsmischung eingesetzt, welche erhalten wurde aus der Umsetzung von Formaldehyd, gegebenenfalls in Form von Paraformaldehyd oder Trioxan, mit mindestens einem Aldehyd der Formel (IV), 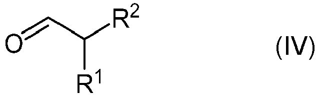 in Gegenwart eines basischen Katalysators mit einem pKa-Wert der konjugierten Säure von mindestens 8. Formaldehyd wird bevorzugt als Formalin oder in Form von Paraformaldehyd eingesetzt, besonders bevorzugt in Form von Paraformaldehyd. Als Aldehyd der Formel (IV) bevorzugt ist Isobutyraldehyd, 2-Methylbutyraldehyd, 2-Ethylbutyraldehyd, 2-Methylvaleraldehyd oder 2-Ethylcapronaldehyd. Besonders bevorzugt ist Isobutyraldehyd.

Diese Reaktionsmischung enthaltend das Aldol der Formel (III) ist frei von starken Säuren, insbesondere halogenhaltigen Säuren wie Bortrichlorid, Bortribromid oder Salzsäure. Somit tritt keine Salzbildung mit dem basischen Katalysator auf, was dessen Aktivität stören würde.

Die Umsetzung von Formaldehyd mit mindestens einem Aldehyd der Formel (IV) ist eine gekreuzte Aldolreaktion. Sie wird in Gegenwart eines basischen Katalysators mit einem pKa-Wert der konjugierten Säure von mindestens 8, bevorzugt mindestens 9, insbesondere mindestens 10, durchgeführt. Bevorzugt handelt es sich dabei um denselben basischen Katalysator, wie er bei der Veresterungsreaktion des Carbonsäureanhydrids der Formel (II) mit dem Aldol der Formel (III), d.h. im erfindungsgemässen Verfahren zur Herstellung eines Aldolesters der Formel (I), eingesetzt wird. Als basischer Katalysator für beide Reaktionen besonders bevorzugt ist Triethylamin.

Der basische Katalysator für die Aldolreaktion wird bevorzugt in einer Menge im Bereich von 0.1 bis 20 Gewichts-%, insbesondere 0.5 bis 15 Gewichts-%, bezogen auf die gesamte Reaktionsmischung der Aldolreaktion eingesetzt. Bevorzugt wird die Aldolreaktion bei einer Temperatur im Bereich von 60 bis 90°C durchgeführt.

Der Aldehyd der Formel (IV) wird bevorzugt im stöchiometrischen Überschuss in Bezug auf Formaldehyd eingesetzt.

Bei der Aldolreaktion kann ein Lösemittel vorhanden sein. Bevorzugt wird die Aldolreaktion ohne organische Lösemittel durchgeführt.

Bevorzugt werden im Anschluss an die Aldolreaktion leichtsiedende Anteile aus der Reaktionsmischung entfernt, insbesondere nicht umgesetzter Aldehyd der Formel (IV), Lösemittel und gegebenenfalls ein Teil des basischen Katalysators, insbesondere mittels Destillation unter Vakuum.

Besonders bevorzugt wird das erfindungsgemässe Verfahren in zwei Stufen durchgeführt, wobei
(i) in der ersten Stufe (Aldolreaktion) der basische Katalysator und Formaldehyd, insbesondere in Form von Paraformaldehyd, vorgelegt werden, dann mindestens ein Aldehyd der Formel (IV) im stöchiometrischen Überschuss in Bezug auf Formaldehyd bei einer Temperatur im Bereich von 60 bis 90°C unter Bildung des Aldols der Formel (III) zugegeben wird und anschliessend leichtsiedende Anteile, insbesondere überschüssiger Aldehyd der Formel (IV) und gegebenenfalls ein Teil des basischen Katalysators, aus der Reaktionsmischung entfernt werden, und
(ii) in der zweiten Stufe (Veresterung) die so erhaltene Reaktionsmischung mit dem Carbonsäureanhydrid der Formel (II) bei einer Temperatur im Bereich von 100 bis 130°C umgesetzt wird, wobei während und/oder nach der Umsetzung leichtsiedende Anteile, insbesondere die aus dem Carbonsäureanhydrid freigesetzte Carbonsäure, nicht umgesetztes Carbonsäureanhydrid und gegebenenfalls der basische Katalysator, aus der Reaktionsmischung entfernt werden, insbesondere mittels Destillation unter Vakuum.

Ein weiterer Gegenstand der Erfindung ist das aus dem erfindungsgemässen Verfahren erhaltene Reaktionsprodukt, insbesondere das aus dem bevorzugten zweistufigen Verfahren erhaltene Reaktionsprodukt, dadurch gekennzeichnet, dass es 60 bis 95 Gewichts-%, insbesondere 65 bis 90 Gewichts-%, besonders bevorzugt 70 bis 85 Gewichts-%, Aldolester der Formel (I) und 5 bis 40 Gewichts-%, bevorzugt 10 bis 35 Gewichts-%, insbesondere 15 bis 30 Gewichts-%, andere, nicht der Formel (I) entsprechende Ester, Aldehyde und/oder Acetale enthält.

Der im Reaktionsprodukt enthaltene Aldolester der Formel (I) ist bevorzugt ausgewählt aus der Gruppe bestehend aus 2,2-Dimethyl-3-acetoxypropanal, 2,2-Dimethyl-3-propionoxypropanal, 2,2-Dimethyl-3-hexanoyloxypropanal, 2,2-Dimethyl-3-(2-ethylhexanoyloxy)propanal und 2,2-Dimethyl-3-benzoyloxypropanal. Besonders bevorzugt ist 2,2-Dimethyl-3-acetoxypropanal.

Bevorzugt enthält das erfindungsgemässe Reaktionsprodukt neben dem Aldolester der Formel (I) Triester der Formel (V) und/oder Acetale der Formel (VI).

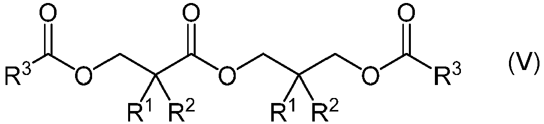

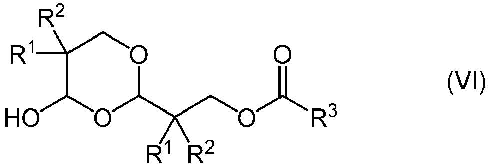

In den Formeln (V) und (VI) weisen R¹, R² und R³ die bereits genannten Bedeutungen auf.

Bevorzugt enthält das erfindungsgemässe Reaktionsprodukt 0.1 bis 20 Gewichts-%, insbesondere 0.5 bis 15 Gewichts-%, besonders bevorzugt 1 bis 10 Gewichts-%, Triester der Formel (V).

Bevorzugt enthält das erfindungsgemässe Reaktionsprodukt 1 bis 20 Gewichts-%, insbesondere 2 bis 15 Gewichts-%, besonders bevorzugt 3 bis 10 Gewichts-%, Acetale der Formel (VI).

Das erfindungsgemässe Reaktionsprodukt weist den Vorteil auf, dass es frei von Halogeniden ist und somit nicht durch aufwendige Aufarbeitungsprozesse von diesen befreit werden muss.

Das erfindungsgemässe Reaktionsprodukt ist klar, hellfarbig und geruchsarm. Es kann dadurch auch ohne weitere Aufreinigung verwendet werden. Thermisch ist das Reaktionsprodukt sehr stabil und zeigt beim Aufheizen bis 200 °C keine nennenswerte Exothermie. Dies ermöglicht eine hohe Prozesssicherheit bei dessen Herstellung und Verarbeitung.

Das erfindungsgemässe Reaktionsprodukt kann vor seiner Verwendung zur Isolierung des Aldolesters der Formel (I) weiter aufgereinigt werden, insbesondere mittels Destillation über Kopf. Dabei ist die hohe thermische Stabilität des Reaktionsprodukts besonders vorteilhaft.

Bevorzugt wird das erfindungsgemässe Reaktionsprodukt ohne weitere Aufreinigung verwendet.

Das erfindungsgemässe Reaktionsprodukt ist geeignet für eine Vielzahl von Verwendungen, insbesondere für die Herstellung von Duftstoffen, Farbstoffen oder Polymeren. Besonders geeignet ist das erfindungsgemässe Reaktionsprodukt als Blockierungsmittel für primäre Amine.

Bevorzugt wird das erfindungsgemässe Reaktionsprodukt für die Herstellung von blockierten Aminen verwendet. Dafür wird das Reaktionsprodukt mit mindestens einem primären Amin umgesetzt. Bei der Umsetzung reagieren die primären Aminogruppen mit den Aldehydgruppen in einer Kondensationsreaktion unter Freisetzung von Wasser und bilden dabei Aldimingruppen, welche eine blockierte, hydrolytisch aktivierbare Form der primären Aminogruppen darstellen.

Die aus der Umsetzung des erfindungsgemässen Reaktionsprodukts mit primären Aminen erhaltenen blockierten Amine sind vorteilhaft als latente Härter in feuchtigkeitshärtenden Polyurethan-Zusammensetzungen verwendbar.

Für die Umsetzung mit dem erfindungsgemässen Reaktionsprodukt bevorzugt sind primäre Amine, welche gegenüber Isocyanatgruppen difunktionell sind, also primäre Amine, welche neben einer primären Aminogruppe zusätzlich mindestens eine weitere primäre Aminogruppe und/oder mindestens eine sekundäre Aminogruppe und/oder mindestens eine Hydroxylgruppe aufweisen. Die daraus erhaltenen blockierten Amine sind besonders geeignet als latente Härter für Polyurethan-Zusammensetzungen. Diese weisen besonders vorteilhafte Eigenschaften in Bezug auf Lagerstabilität, Verarbeitbarkeit, Aushärtung und mechanischen Eigenschaften auf.

Ein weiterer Gegenstand der Erfindung ist somit ein blockiertes Amin, erhalten aus der Umsetzung des erfindungsgemässen Reaktionsprodukts mit mindestens einem Amin, welches eine primäre Aminogruppe und zusätzlich mindestens eine Reaktivgruppe ausgewählt aus primärer Aminogruppe, sekundärer Aminogruppe und Hydroxylgruppe aufweist. Bevorzugt enthält das Amin nur eine sekundäre Aminogruppe oder nur eine Hydroxylgruppe. Besonders bevorzugt ist das Amin frei von sekundären Aminogruppen.

Ein so erhaltenes blockiertes Amin enthält zusätzlich zum Aldimin aus der Umsetzung des Aldolesters der Formel (I) die im eingesetzten Reaktionsprodukt enthaltenen Nebenprodukte aus dem erfindungsgemässen Verfahren, insbesondere die beschriebenen Triester der Formel (V) und/oder Acetale der Formel (VI), und/oder deren Umsetzungsprodukte mit dem Amin.

Geeignete Amine zum Blockieren sind insbesondere
- primäre aliphatische Diamine wie insbesondere 1,2-Ethandiamin, 1,2-Propandiamin, 1,3-Propandiamin, 1,4-Butandiamin, 1,3-Butandiamin, 2-Methyl-1,2-propandiamin, 1,3-Pentandiamin, 1,5-Pentandiamin, 2,2-Dimethyl-1,3-propandiamin, 1,6-Hexandiamin, 1,5-Diamino-2-methylpentan, 1,7-Heptandiamin, 1,8-Octandiamin, 2,5-Dimethyl-1,6-hexandiamin, 1,9-Nonandiamin, 2,2(4),4-Trimethyl-1,6-hexandiamin, 1,10-Decandiamin, 1,11-Undecandiamin, 2-Butyl-2-ethyl-1,5-pentandiamin, 1,12-Dodecandiamin, 1,2-Cyclohexandiamin, 1,3-Cyclohexandiamin, 1,4-Cyclohexandiamin, 1-Amino-3-aminomethyl-3,5,5-trimethylcyclohexan, 4(2)-Methyl-1,3-cyclohexandiamin, 1,3-Bis(aminomethyl)-cyclohexan, 1,4-Bis(aminomethyl)cyclohexan, Bis(4-aminocyclohexyl)methan, Bis(4-amino-3-methylcyclohexyl)methan, Bis(4-amino-3-ethylcyclohexyl)methan, Bis-(4-amino-3,5-dimethylcyclohexyl)methan, Bis(4-amino-3-ethyl-5-methylcyclohexyl)methan, 2,5(2,6)-Bis(aminomethyl)bicyclo[2.2.1]heptan, 3(4),8(9)-Bis(aminomethyl)tricyclo[5.2.1.0^{2,6}]decan, 1,3-Bis(aminomethyl)benzol, 1,4-Bis(aminomethyl)benzol, 3-Oxa-1,5-pentandiamin, 3,6-Dioxaoctan-1,8-diamin, 4,7-Dioxadecan-1,10-diamin, 4,7-Dioxadecan-2,9-diamin, 4,9-Dioxado-decan-1,12-diamin, 5,8-Dioxadodecan-3,10-diamin, 4,7,10-Trioxatridecan-1,13-diamin, α,ω-Polyoxypropylendiamine mit einem mittleren Molekulargewicht Mₙ im Bereich von 170 bis 4'000 g/mol, insbesondere die Jeffamine^{®}-Typen D-230, D-400, XTJ-582, D-2000, XTJ-578, D-4000 (alle von Huntsman), α,ω-Polyoxy-propylenpolyoxyethylendiamin, insbesondere die Jeffamine^{®}-Typen ED-600, ED-900, ED-2003, HK-511 (alle von Huntsman), α,ω-Polyoxypropylenpolyoxy-1,4-butylendiamin, insbesondere die Jeffamine^{®}-Typen THF-100, THF-140, THF-230, XTJ-533 oder XTJ-536 (alle von Huntsman), α,ω-Polyoxypropylen-polyoxy-1,2-butylendiamin, insbesondere die Jeffamine^{®}-Typen XTJ-568 oder XTJ-569 (beide von Huntsman) oder α,ω-Polyoxy-1,2-butylendiamin, insbesondere Jeffamine^{®} XTJ-523 (von Huntsman),
- primäre aliphatische Triamine wie insbesondere 1,3,6-Triaminohexan, 1,4,8-Triaminooctan, 4-Aminomethyl-1,8-octandiamin, 5-Aminomethyl-1,8-octan-diamin, 1,6,11-Triaminoundecan, 1,3,5-Triaminocyclohexan, 1,3,5-Tris-(aminomethyl)cyclohexan, 1,3,5-Tris(aminomethyl)benzol, Trimethylolpropan- oder Glycerin-gestartetes Tris(ω-polyoxypropylenamin) mit einem mittleren Molekulargewicht Mₙ im Bereich von 330 bis 6'000 g/mol, insbesondere die Jeffamine^{®}-Typen T-403, T-3000 oder T-5000 (alle von Huntsman), oder Trimethylolpropan-gestartetes Tris(ω-polyoxypropylen-polyoxy-1,2-butylenamin), insbesondere Jeffamine^{®} XTJ-566 (von Huntsman), oder
- primäre aromatische Diamine wie insbesondere 1,3-Phenylendiamin, 1,4-Phenylendiamin, 4(2)-Methyl-1,3-phenylendiamin (TDA), 3,5-Diethyl-2,4(6)-toluylendiamin (DETDA) oder 4,4'-Diaminodiphenylmethan (MDA), oder
- aliphatische Diamine mit einer primären und einer sekundären Aminogruppe wie insbesondere N-Methyl-1,2-ethandiamin, N-Ethyl-1,2-ethandiamin, N-Butyl-1,2-ethandiamin, N-Hexyl-1,2-ethandiamin, N-(2-Ethylhexyl)-1,2-ethandiamin, N-Cyclohexyl-1,2-ethandiamin, N-Benzyl-1,2-ethandiamin, 4-Aminomethylpiperidin, 3-(4-Aminobutyl)piperidin, N-(2-Aminoethyl)piperazin, N-(2-Amino-propyl)piperazin, N-Benzyl-1,2-propandiamin, N, Benzyl-1,3-propandiamin, N-Methyl-1,3-propandiamin, N-Ethyl-1,3-propandiamin, N-Butyl-1,3-propandiamin, N-Hexyl-1,3-propandiamin, N-(2-Ethylhexyl)-1,3-propandiamin, N-Dodecyl-1,3-propandiamin, N-Cyclohexyl-1,3-propandiamin, 3-Methylamino-1-pentylamin, 3-Ethylamino-1-pentylamin, 3-Butylamino-1-pentylamin, 3-Hexylamino-1-pentyl-amin, 3-(2-Ethylhexyl)amino-1-pentylamin, 3-Dodecylamino-1-pentylamin, 3-Cyclohexylamino-1-pentylamin, Fettdiamine wie N-Cocoalkyl-1,3-propandiamin, N-Oleyl-1,3-propandiamin, N-Soyaalkyl-1,3-propandiamin, N-Talgalkyl-1,3-propandiamin oder N-(C₁₆₋₂₂-Alkyl)-1,3-propandiamin, wie sie beispielsweise unter dem Handelsnamen Duomeen^{®} von Akzo Nobel erhältlich sind, oder Produkte aus der Michael-artigen Addition von aliphatischen primären Diaminen mit Acrylnitril, Malein- oder Fumarsäurediestern, Citraconsäurediestern, (Meth)-acrylsäureestern, (Meth)acrylsäureamiden oder Itaconsäurediestern, umgesetzt im Molverhältnis 1:1, oder
- aliphatische Polyamine mit zwei primären und einer sekundären Aminogruppe wie insbesondere Bis(hexamethylen)triamin (BHMT), Diethylentriamin (DETA), Dipropylentriamin (DPTA), N-(2-Aminoethyl)-1,3-propandiamin (N3-Amin), N3-(3-Aminopentyl)-1,3-pentandiamin oder N5-(3-Amino-1-ethylpropyl)-2-methyl-1,5-pentandiamin, oder
- Hydroxylamine wie insbesondere 2-Aminoethanol, 2-Amino-1-propanol, 1-Amino-2-propanol, 3-Amino-1-propanol, 4-Amino-1-butanol, 4-Amino-2-butanol, 2-Amino-2-methylpropanol, 5-Amino-1-pentanol, 6-Amino-1-hexanol, 7-Amino-1-heptanol, 8-Amino-1-octanol, 10-Amino-1-decanol, 12-Amino-1-dodecanol oder höhere Homologe davon, 4-(2-Aminoethyl)-2-hydroxyethylbenzol, 3-Aminomethyl-3,5,5-trimethylcyclohexanol, eine primäre Aminogruppe tragende Derivate von Glykolen wie Diethylenglykol, Dipropylenglykol, Dibutylenglykol oder höhere Oligomere oder Polymere dieser Glykole, insbesondere 2-(2-Aminoethoxy)ethanol, 2-(2-(2-Aminoethoxy)ethoxy)ethanol oder α-(2-Hydroxymethylethyl)-ω-(2-aminomethylethoxy)-poly(oxy(methyl-1,2-ethandiyl), 3-(2-Hydroxyethoxy)propylamin, 3-(2-(2-Hydroxyethoxy)ethoxy)propylamin oder 3-(6-Hydroxyhexyloxy)-propylamin.

Insbesondere ist das Amin ausgewählt aus der Gruppe bestehend aus 1,6-Hexandiamin, 1-Amino-3-aminomethyl-3,5,5-trimethylcyclohexan, 4(2)-Methyl-1,3-cyclohexandiamin, 1,3-Bis(aminomethyl)cyclohexan, 1,4-Bis(aminomethyl)cyclohexan, 1,3-Bis(aminomethyl)benzol, 1,2-Cyclohexandiamin, 1,3-Cyclohexandiamin, 1,4-Cyclohexandiamin, Bis(4-aminocyclohexyl)methan, 2,5(2,6)-Bis(aminomethyl)-bicyclo[2.2.1]heptan, 3(4),8(9)-Bis(aminomethyl)tricyclo[5.2.1.0^{2,6}]decan, α,ω-Polyoxypropylendiamin mit einem mittleren Molekulargewicht Mₙ im Bereich von 170 bis 500 g/mol, insbesondere die Jeffamine^{®}-Typen D-230 oder D-400 (von Huntsman), Trimethylolpropan- oder Glycerin-gestartetes Tris(ω-polyoxypropylenamin) mit einem mittleren Molekulargewicht Mₙ im Bereich von 330 bis 500 g/mol, insbesondere Jeffamine^{®} T-403 (von Huntsman), 1,4-Phenylendiamin, 3,5-Diethyl-2,4(6)-toluylendiamin, 2-(2-Aminoethoxy)ethanol, 2-(2-(2-Aminoethoxy)ethoxy)-ethanol und 3-Aminomethyl-3,5,5-trimethylcyclohexanol.

Davon bevorzugt ist 1,6-Hexandiamin, 1-Amino-3-aminomethyl-3,5,5-trimethylcyclohexan, α,ω-Polyoxypropylendiamin mit einem mittleren Molekulargewicht Mₙ im Bereich von 170 bis 300 g/mol, Trimethylolpropan-gestartetes Tris(ω-polyoxy-propylenamin) mit einem mittleren Molekulargewicht Mₙ im Bereich von 330 bis 500 g/mol oder 2-(2-Aminoethoxy)ethanol.

Die bevorzugten Amine sind einfach zugänglich. Sie ermöglichen in blockierter Form feuchtigkeitshärtende Polyurethan-Zusammensetzungen mit guter Lagerstabilität, guter Verarbeitbarkeit, schneller Aushärtung und hoher Festigkeit bei hoher Dehnbarkeit.

Für den Fall, dass das blockierte Amin eine Hydroxylgruppe oder eine sekundäre Aminogruppe aufweist, reagiert diese Gruppe bei der Lagerung mit vorhandenen Isocyanatgruppen.

Das erfindungsgemässe blockierte Amin wird bevorzugt hergestellt, indem
- das erfindungsgemässe Reaktionsprodukt mit dem Amin zu einer Reaktionsmischung vereint wird, gegebenenfalls unter Einsatz eines Lösemittels, wobei die Aldehydgruppen gegenüber den primären Aminogruppen stöchiometrisch oder im stöchiometrischen Überschuss vorhanden sind, und
- das bei der Reaktion entstehende Kondensationswasser und gegebenenfalls eingesetztes Lösemittel während oder nach der Vereinigung mit einer geeigneten Methode aus der Reaktionsmischung entfernt werden.

Bevorzugt wird das Kondensationswasser und gegebenenfalls eingesetztes Lösemittel mittels Anlegen von Vakuum aus der erwärmten Reaktionsmischung entfernt.

Bevorzugt wird kein Lösemittel eingesetzt.

Die Umsetzung wird bevorzugt bei einer Temperatur im Bereich von 20 bis 120°C, insbesondere bei 40 bis 100°C, durchgeführt.

Gegebenenfalls wird bei der Umsetzung ein Katalysator eingesetzt, insbesondere ein Säure-Katalysator.

Das erfindungsgemässe blockierte Amin enthält insbesondere mindestens ein Aldimin der Formel (VII), 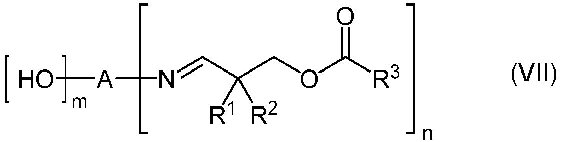 wobei
m für 0 oder 1 steht, n für 1, 2 oder 3 steht und (m+n) für 2 oder 3 steht,
A für einen (m+n)-wertigen organischen Rest mit 2 bis 25 C-Atomen steht, und
R¹, R² und R³ die bereits beschriebenen Bedeutungen aufweisen.

Bevorzugt stehen m für 0 und n für 2 oder 3. Ein solches Aldimin der Formel (VII) ist ein Di- oder Trialdimin.

Weiterhin bevorzugt stehen m für 1 und n für 1. Ein solches Aldimin der Formel (VII) ist ein Hydroxyaldimin.

Bevorzugt steht A für einen gegebenenfalls cyclische Anteile aufweisenden Alkylen-Rest oder für einen zwei- oder dreiwertigen Polyoxyalkylen-Rest mit 5 bis 15 C-Atomen.

Besonders bevorzugt steht A für einen Rest ausgewählt aus der Gruppe bestehend aus 1,6-Hexylen, (1,5,5-Trimethylcyclohexan-1-yl)methan-1,3, α,ω-Polyoxypropylen mit einem mittleren Molekulargewicht Mₙ im Bereich von 170 bis 300 g/mol, Trimethylolpropan-gestartetes Tris(ω-polyoxypropylen) mit einem mittleren Molekulargewicht Mₙ im Bereich von 330 bis 500 g/mol, 1,4-Phenylen, 3,5-Diethyl-2,4(6)-toluylen und 3-Oxa-1,5-pentylen.

Das Aldimin der Formel (VII) ist besonders bevorzugt ausgewählt aus der Gruppe bestehend aus N,N'-Bis(2,2-dimethyl-3-acetoxypropyliden)hexylen-1,6-diamin, N,N'-Bis(2,2-dimethyl-3-acetoxypropyliden)-3-aminomethyl-3,5,5-trimethylcyclohexylamin, N,N'-Bis(2,2-dimethyl-3-acetoxypropyliden)polyoxypropylendiamin mit einem mittleren Molekulargewicht Mₙ im Bereich von 450 bis 880 g/mol, N,N',N"-Tris(2,2-dimethyl-3-acetoxypropyliden)polyoxypropylentriamin mit einem mittleren Molekulargewicht Mₙ im Bereich von 730 bis 880 g/mol, N,N'-Bis(2,2-dimethyl-3-acetoxypropyliden)phenylen-1,4-diamin, N,N'-Bis(2,2-dimethyl-3-acetoxypropyliden)-3,5-diethyl-toluylen-2,4(6)-diamin und N-(2,2-Dimethyl-3-acetoxypropyliden)-2-(2-aminoethoxy)ethan-1 -ol.

Die bevorzugten blockierten Amine ermöglichen feuchtigkeitshärtende Polyurethan-Zusammensetzungen mit guter Lagerstabilität, guter Verarbeitbarkeit, besonders schneller Aushärtung und besonders hoher Festigkeit bei hoher Dehnbarkeit. Im Fall von N-(2,2-Dimethyl-3-acetoxypropyliden)-2-(2-amino-ethoxy)ethan-1-ol reagiert die Hydroxylgruppe während der Lagerung mit vorhandenen Isocyanatgruppen.

Ein weiterer Gegenstand der Erfindung ist eine feuchtigkeitshärtende Polyurethan-Zusammensetzung enthaltend
- mindestens ein Polyisocyanat und/oder Isocyanatgruppen-haltiges Polymer und
- mindestens ein blockiertes Amin aus der Umsetzung des erfindungsgemässen Reaktionsprodukts, wie vorgängig beschrieben.

Bevorzugt enthält die feuchtigkeitshärtende Polyurethan-Zusammensetzung ein blockiertes Amin enthaltend mindestens ein Aldimin der Formel (VII).

Als Polyisocyanat geeignet sind
- handelsübliche aromatische, aliphatische oder cycloaliphatische Diisocyanate, wie insbesondere 4,4'-Diphenylmethandiisocyanat, gegebenenfalls mit Anteilen von 2,4'- und/oder 2,2'-Diphenylmethandiisocyanat (MDI), 2,4'-Diphenylmethandiisocyanat (2,4'-MDI), 2,4-Toluylendiisocyanat oder Gemische davon mit 2,6-Toluylendiisocyanat (TDI), 1,4-Phenylendiisocyanat (PDI), Naphthalin-1,5-diisocyanat (NDI), 1,6-Hexandiisocyanat (HDI), 2,2(4),4-Trimethyl-1,6-hexamethylendiisocyanat (TMDI), Cyclohexan-1,3- oder -1,4-diisocyanat, 1-Isocyanato-3,3,5-trimethyl-5-isocyanatomethylcyclohexan (Isophorondiisocyanat oder IPDI), Perhydro-2,4'- oder -4,4'-diphenylmethandiisocyanat (HMDI), 1,3- oder 1,4-Bis(isocyanatomethyl)cyclohexan, m- oder p-Xylylendiisocyanat (XDI), oder Gemische davon,
- höherfunktionelle Derivate solcher Diisocyanate, insbesondere durch Carbodiimidisierung bzw. Uretoniminbildung oder Adduktbildung mit Polyolen verflüssigtes 4,4'-Diphenylmethandiisocyanat,
- ein bei Raumtemperatur flüssiges Gemisch von MDI mit MDI-Homologen (polymeres MDI oder PMDI,
- Diisocyanat-Oligomere, wie insbesondere HDI-Biurete wie Desmodur^{®} N 100 oder N 3200 (von Covestro), Tolonate^{®} HDB oder HDB-LV (von Vencorex) oder Duranate^{®} 24A-100 (von Asahi Kasei); HDI-Isocyanurate wie Desmodur^{®} N 3300, N 3600 oder N 3790 BA (alle von Covestro), Tolonate^{®} HDT, HDT-LV oder HDT-LV2 (von Vencorex), Duranate^{®} TPA-100 oder THA-100 (von Asahi Kasei) oder Coronate^{®} HX (vonTosoh Corp.); HDI-Uretdione wie Desmodur^{®} N 3400 (von Covestro); HDI-Iminooxadiazindione wie Desmodur^{®} XP 2410 (von Covestro); HDI-Allophanate wie Desmodur^{®} VP LS 2102 (von Covestro); IPDI-Isocyanurate wie beispielsweise in Lösung als Desmodur^{®} Z 4470 (von Covestro) oder in fester Form als Vestanat^{®} T1890/ 100 (von Evonik Industries); TDI-Oligomere wie Desmodur^{®} IL (von Covestro); oder gemischte Isocyanurate auf Basis TDI/HDI wie Desmodur^{®} HL (von Covestro); oder
- handelsüblicheTriisocyanate, wie insbesondere 4,4',4"-Triphenylmethantriisocyanat, erhältlich als Desmodur^{®} RE (von Covestro), oder Tris(p-isocyanatophenyl)thiophospat, erhältlich als Desmodur^{®} RFE (von Covestro).

Ein geeignetes Isocyanatgruppen-haltiges Polymer ist insbesondere ein Umsetzungsprodukt von mindestens einem Polyol mit einer überstöchiometrischen Menge von mindestens einem Diisocyanat. Die Umsetzung wird bevorzugt unter Feuchtigkeitsausschluss bei einer Temperatur im Bereich von 20 bis 160 °C, insbesondere 40 bis 140 °C, durchgeführt, gegebenenfalls in Anwesenheit geeigneter Katalysatoren.

Das NCO/OH-Verhältnis liegt bevorzugt im Bereich von 1.3/1 bis 10/1. Das nach der Umsetzung der OH-Gruppen im Reaktionsgemisch verbleibende monomere Diisocyanat kann entfernt werden, insbesondere mittels Destillation.

Für den Fall, dass monomeres Diisocyanat aus dem Polymer entfernt wird, liegt das NCO/OH-Verhältnis bei der Umsetzung bevorzugt im Bereich von 3/1 bis 10/1, insbesondere 4/1 bis 7/1, und das erhaltene Isocyanatgruppen-haltige Polymer enthält nach der Destillation bevorzugt höchstens 0.5 Gewichts-%, besonders bevorzugt höchstens 0.3 Gewichts-%, monomeres Diisocyanat. Dabei wird monomeres Diisocyanat insbesondere mittels Kurzwegdestillation im Vakuum entfernt. Für den Fall, dass monomeres Diisocyanat nicht aus dem Polymer entfernt wird, liegt das NCO/OH-Verhältnis bei der Umsetzung bevorzugt im Bereich von 1.3/1 bis 2.5/1. Ein solches Polyetherurethan-Polymer enthält insbesondere höchstens 3 Gewichts-%, bevorzugt höchstens 2 Gewichts-%, monomeres Diisocyanat.

Als monomeres Diisocyanat bevorzugt sind die bereits genannten aromatischen, aliphatischen oder cycloaliphatischen Diisocyanate, insbesondere MDI, TDI, HDI, HMDI oder IPDI, oder Gemische davon.

Besonders bevorzugt ist 4,4'-MDI, TDI oder IPDI.

Geeignete Polyole sind handelsübliche Polyole oder Mischungen davon, insbesondere
- Polyetherpolyole, insbesondere Polyoxyalkylendiole und/oder Polyoxyalkylentriole, insbesondere Polymerisationsprodukte von Ethylenoxid oder 1,2-Propylenoxid oder 1,2- oder 2,3-Butylenoxid oder Oxetan oder Tetrahydrofuran oder Mischungen davon, wobei diese mit Hilfe eines Startermoleküls mit zwei oder drei aktiven Wasserstoffatomen polymerisiert sein können, insbesondere einem Startermolekül wie Wasser, Ammoniak oder einer Verbindung mit mehreren OH- oder NH-Gruppen wie beispielsweise 1,2-Ethandiol, 1,2- oder 1,3-Propandiol, Neopentylglykol, Diethylenglykol, Triethylenglykol, die isomeren Dipropylenglykole oder Tripropylenglykole, die isomeren Butandiole, Pentandiole, Hexandiole, Heptandiole, Octandiole, Nonandiole, Decandiole, Undecandiole, 1,3- oder 1,4-Cyclohexandimethanol, Bisphenol A, hydriertes Bisphenol A, 1,1,1-Trimethylolethan, 1,1,1-Trimethylolpropan, Glycerin oder Anilin, oder Mischungen der vorgenannten Verbindungen. Ebenfalls geeignet sind Polyetherpolyole mit darin dispergierten Polymerpartikeln, insbesondere solche mit Styrol-Acrylnitril-Partikeln (SAN) oder Polyharnstoff- bzw. Polyhydrazodicarbonamid-Partikeln (PHD). Bevorzugte Polyetherpolyole sind Polyoxypropylendiole oder Polyoxypropylentriole, oder sogenannte Ethylenoxid-terminierte (EO-capped bzw. EO-tipped) Polyoxypropylendiole oder -triole. Letztere sind Polyoxyethylen-polyoxypropylen-Mischpolyole, die insbesondere dadurch erhalten werden, dass Polyoxypropylendiole oder -triole nach Abschluss der Polypropoxylierungsreaktion mit Ethylenoxid weiter alkoxyliert werden und dadurch primäre Hydroxylgruppen aufweisen.
   Bevorzugte Polyetherpolyole weisen einen Ungesättigtheitsgrad von weniger als 0.02 mEq/g, insbesondere weniger als 0.01 mEq/g auf.
- Polyesterpolyole, auch Oligoesterole genannt, hergestellt nach bekannten Verfahren, insbesondere der Polykondensation von Hydroxycarbonsäuren bzw. Lactonen oder der Polykondensation von aliphatischen und/oder aromatischen Polycarbonsäuren mit zwei- oder mehrwertigen Alkoholen. Bevorzugt sind Polyesterdiole aus der Umsetzung von zweiwertigen Alkoholen wie insbesondere 1,2-Ethandiol, Diethylenglykol, 1,2-Propandiol, Dipropylenglykol, 1,4-Butandiol, 1,5-Pentandiol, 1,6-Hexandiol, Neopentylglykol, Glycerin, 1,1,1-Trimethylolpropan oder Mischungen der vorgenannten Alkohole, mit organischen Dicarbonsäuren oder deren Anhydride oder Ester, wie insbesondere Bernsteinsäure, Glutarsäure, Adipinsäure, Korksäure, Sebacinsäure, Dodecandicarbonsäure, Maleinsäure, Fumarsäure, Phthalsäure, Isophthalsäure, Terephthalsäure, 1,2-Cyclohexandicarbonsäure, 1,3-Cyclohexandicarbonsäure oder 1,4-Cyclohexandicarbonsäure oder Mischungen der vorgenannten Säuren, oder Polyesterpolyole aus Lactonen wie insbesondere ε-Caprolacton. Besonders bevorzugt sind Polyesterpolyole aus Adipinsäure oder Sebacinsäure oder Dodecandicarbonsäure und Hexandiol oder Neopentylglykol.
- Polycarbonatpolyole, wie sie durch Umsetzung beispielsweise der oben genannten - zum Aufbau der Polyesterpolyole eingesetzten - Alkohole mit Dialkylcarbonaten, Diarylcarbonaten oder Phosgen zugänglich sind.
- Mindestens zwei OH-Gruppen tragende Blockcopolymere, welche mindestens zwei verschiedene Blöcke mit Polyether-, Polyester- und/oder Polycarbonatstruktur der oben beschriebenen Art aufweisen, insbesondere Polyetherpolyesterpolyole.
- Polyacrylat- oder Polymethacrylatpolyole.
- Polyhydroxyfunktionelle Fette oder Öle, beispielsweise natürliche Fette und Öle, inbesondere Ricinusöl; oder durch chemische Modifizierung von natürlichen Fetten und Ölen gewonnene - sogenannte oleochemische - Polyole, beispielsweise die durch Epoxidierung ungesättigter Öle und anschliessender Ringöffnung mit Carbonsäuren bzw. Alkoholen erhaltenen Epoxypolyester bzw. Epoxypolyether, oder durch Hydroformylierung und Hydrierung ungesättigter Öle erhaltene Polyole; oder aus natürlichen Fetten und Ölen durch Abbauprozesse wie Alkoholyse oder Ozonolyse und anschliessender chemischer Verknüpfung, beispielsweise durch Umesterung oder Dimerisierung, der so gewonnenen Abbauprodukte oder Derivaten davon erhaltene Polyole. Geeignete Abbauprodukte von natürlichen Fetten und Ölen sind insbesondere Fettsäuren und Fettalkohole sowie Fettsäureester, insbesondere die Methylester (FAME), welche beispielsweise durch Hydroformylierung und Hydrierung zu Hydroxyfettsäureestern derivatisiert werden können.
- Polykohlenwasserstoffpolyole, auch Oligohydrocarbonole genannt, wie insbesondere polyhydroxyfunktionelle Polyolefine, Polyisobutylene, Polyisoprene; polyhydroxyfunktionelle Ethylen-Propylen-, Ethylen-Butylen- oder Ethylen-Propylen-Dien-Copolymere, wie sie beispielsweise von der Firma Kraton Polymers hergestellt werden; polyhydroxyfunktionelle Polymere von Dienen, insbesondere von 1,3-Butadien, welche insbesondere auch aus anionischer Polymerisation hergestellt sein können; polyhydroxyfunktionelle Copolymere aus Dienen wie 1,3-Butadien oder Diengemischen und Vinylmonomeren wie Styrol, Acrylonitril, Vinylchlorid, Vinylacetat, Vinylalkohol, Isobutylen oder Isopren, insbesondere polyhydroxyfunktionelle Acrylonitril/ Butadien-Copolymere, wie sie insbesondere aus Epoxiden oder Aminoalkoholen und carboxylterminierten Acrylonitril/Butadien-Copolymeren (beispielsweise kommerziell erhältlich unter dem Namen Hypro^{®} CTBN oder CTBNX oder ETBN von Emerald Performance Materials) hergestellt werden können; oder hydrierte polyhydroxyfunktionelle Polymere oder Copolymere von Dienen.

Geeignet sind insbesondere auch Mischungen von Polyolen.

Bevorzugt sind Polyetherpolyole, Polyesterpolyole, Polycarbonatpolyole, Poly-(meth)acrylatpolyole oder Polybutadienpolyole.

Besonders bevorzugt sind Polyetherpolyole, Polyesterpolyole, insbesondere aliphatische Polyesterpolyole, oder Polycarbonatpolyole, insbesondere aliphatische Polycarbonatpolyole.

Insbesondere bevorzugt sind Polyetherpolyole, insbesondere Polyoxyalkylenpolyole.

Am meisten bevorzugt sind Polyoxypropylendi- oder -triole oder Ethylenoxid-terminierte Polyoxypropylendi- oder -triole.

Bevorzugt sind Polyole mit einem mittleren Molekulargewicht Mₙ im Bereich von 400 bis 20'000 g/mol, bevorzugt von 1'000 bis 15'000 g/mol.

Bevorzugt sind Polyole mit einer mittleren OH-Funktionalität im Bereich von 1.6 bis 3.

Bevorzugt sind bei Raumtemperatur flüssige Polyole.

Bei der Herstellung eines Isocyanatgruppen-haltigen Polymers können auch Anteile von zwei- oder mehrfunktionellen Alkoholen mitverwendet werden, insbesondere 1,2-Ethandiol, 1,2-Propandiol, 1,3-Propandiol, 2-Methyl-1,3-propandiol, 1,2-Butandiol, 1,3-Butandiol, 1,4-Butandiol, 1,3-Pentandiol, 1,5-Pentandiol, 3-Methyl-1,5-pentandiol, Neopentylglykol, Dibromneopentylglykol, 1,2-Hexandiol, 1,6-Hexandiol, 1,7-Heptandiol, 1,2-Octandiol, 1,8-Octandiol, 2-Ethyl-1,3-hexandiol, Diethylenglykol, Triethylenglykol, Dipropylenglykol, Tripropylenglykol, 1,3-oder 1,4-Cyclohexandimethanol, ethoxyliertes Bisphenol-A, propoxyliertes Bisphenol-A, Cyclohexandiol, hydriertes Bisphenol-A, Dimerfettsäurealkohole, 1,1,1-Trimethylolethan, 1,1,1-Trimethylolpropan, Glycerin, Pentaerythritol, Zuckeralkohole wie insbesondere Xylit, Sorbit oder Mannit oder Zucker wie insbesondere Saccharose oder alkoxylierte Derivate der genannten Alkohole oder Mischungen der genannten Alkohole.

Bevorzugt enthält die feuchtigkeitshärtende Polyurethan-Zusammensetzung mindestens ein Isocyanatgruppen-haltiges Polymer.

Das Isocyanatgruppen-haltige Polymer weist bevorzugt ein mittleres Molekulargewicht Mₙ im Bereich von 1'500 bis 20'000 g/mol, insbesondere 2'000 bis 15'000 g/mol, auf.

Das Isocyanatgruppen-haltige Polymer hat bevorzugt einen Gehalt an Isocyanatgruppen im Bereich von 0.5 bis 10 Gewichts-%, insbesondere 1 bis 5 Gewichts-%.

Das Isocyanatgruppen-haltige Polymer hat bevorzugt einen geringen Gehalt an monomerem Diisocyanat, bevorzugt weniger als 2 Gewichts-%, insbesondere weniger als 1 Gewichts-%, monomeres Diisocyanat.

Bevorzugt enthält die feuchtigkeitshärtende Polyurethan-Zusammensetzung zusätzlich mindestens einen weiteren Bestandteil ausgewählt aus Füllstoffen, Weichmachern, weiteren blockierten Aminen, Katalysatoren und Stabilisatoren.

Geeignete Füllstoffe sind insbesondere gemahlene oder gefällte Calciumcarbonate, welche gegebenenfalls mit Fettsäuren, insbesondere Stearaten, beschichtet sind, Baryte (Schwerspate), Quarzmehle, Quarzsande, Dolomite, Wollastonite, calcinierte Kaoline, Schichtsilikate wie Glimmer oder Talk, Zeolithe, Aluminiumhydroxide, Magnesiumhydroxide, Kieselsäuren inklusive hochdisperse Kieselsäuren aus Pyrolyseprozessen, Zemente, Gipse, Flugaschen, industriell hergestellte Russe, Graphit, Metall-Pulver, beispielsweise von Aluminium, Kupfer, Eisen, Silber oder Stahl, PVC-Pulver oder Leichtfüllstoffe wie Glashohlkugeln oder gasgefüllte Kunststoffhohlkugeln (microspheres), insbesondere die unter dem Handelsnamen Expancel^{®} (von Akzo Nobel) erhältlichen Typen.

Bevorzugt sind Calciumcarbonate, welche gegebenenfalls mit Fettsäuren, insbesondere Stearaten, beschichtet sind, calcinierte Kaoline, hochdisperse Kieselsäuren oder industriell hergestellte Russe.

Geeignete Weichmacher sind insbesondere Carbonsäureester wie Phthalate, insbesondere Diisononylphthalat (DINP), Diisodecylphthalat (DIDP) oder Di(2-propylheptyl)phthalat (DPHP), hydrierte Phthalate bzw. 1,2-Cyclohexandicarbonsäureester, insbesondere hydriertes Diisononylphthalat bzw. Diisononyl-1,2-cyclohexandicarboxylat (DINCH), Terephthalate, insbesondere Bis(2-ethylhexyl)terephthalat (DOTP) oder Diisononylterephthalat (DINT), hydrierte Terephthalate bzw. 1,4-Cyclohexandicarbonsäureester, insbesondere hydriertes Bis(2-ethylhexyl)terephthalat bzw. Bis(2-ethylhexyl)-1,4-cyclohexandicarboxylat oder hydriertes Diisononylterephthalat bzw. Diisononyl-1,4-cyclohexandicarboxylat, Isophthalate, Trimellitate, Adipate, insbesondere Dioctyladipat, Azelate, Sebacate, Benzoate, Glykolether, Glykolester, Weichmacher mit Polyetherstruktur, insbesondere Polypropylenoxid-monole, -diole oder -triole mit blockierten Hydroxylgruppen, insbesondere in der Form von Acetatgruppen, organische Phosphor-oder Sulfonsäureester, Polybutene, Polyisobutene oder von natürlichen Fetten oder Ölen abgeleitete Weichmacher, insbesondere epoxidiertes Soja- oder Leinöl. Bevorzugte Weichmacher sind Phthalate, hydrierte Phthalate, Adipate oder Weichmacher mit Polyetherstruktur.

Geeignete weitere blockierte Amine sind insbesondere Oxazolidine oder Aldimine. Bevorzugt als weiteres blockiertes Amin ist ein Bis-Oxazolidin der Formel (VIII) oder (IX), 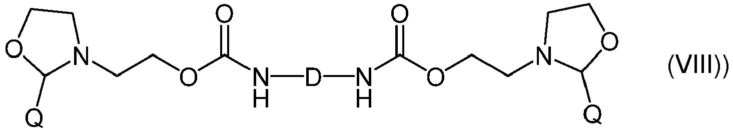 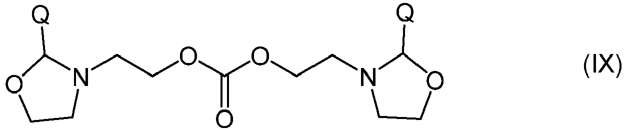 wobei
D für einen zweiwertigen Kohlenwasserstoffrest mit 6 bis 15 C-Atomen, insbesondere für 1,6-Hexylen oder (1,5,5-Trimethylcyclohexan-1-yl)methan-1,3 oder 4(2)-Methyl-1,3-phenylen, steht, und
Q für einen einwertigen organischen Rest mit 3 bis 26 C-Atomen, insbesondere für 2-Propyl, 3-Heptyl, Phenyl oder einen substituierten Phenylrest, insbesondere für einen in para-Stellung mit einem gegebenenfalls verzweigten Decylphenyl-, Undecylphenyl, Dodecylphenyl, Tridecylphenyl- oder Tetradecylphenyl-Rest substituieren Phenylrest, steht.

Weiterhin bevorzugt als weiteres blockiertes Amin ist ein Mono-Oxazolidin der Formel 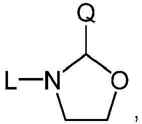 wobei L für einen Alkyl-, Cycloalkyl- oder Arylalkyl-Rest mit 1 bis 8 C-Atomen steht, insbesondere für Methyl, Ethyl oder n-Butyl, und Q die bereits genannten Bedeutungen aufweist.

Weiterhin bevorzugt als weiteres blockiertes Amin ist ein Aldimin der Formel 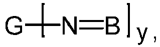 wobei y für 2 oder 3, G für einen organischen Rest mit 2 bis 23 C-Atomen und B für einen organischen Rest mit 6 bis 30 C-Atomen stehen. Bevorzugt steht G für einen gegebenenfalls cyclische Anteile aufweisenden Alkylen-Rest oder einen zwei- oder dreiwertigen Polyoxyalkylen-Rest mit 5 bis 15 C-Atomen, insbesondere für 1,6-Hexylen, (1,5,5-Trimethylcyclohexan-1-yl)methan-1,3 oder α,ω-Polyoxypropylen mit einem mittleren Molekulargewicht Mₙ im Bereich von 170 bis 300 g/mol oder Trimethylolpropan-gestartetes Tris(ω-polyoxypropylen) mit einem mittleren Molekulargewicht Mₙ im Bereich von 330 bis 500 g/mol. Bevorzugt steht B für einen organischen Rest mit 7 bis 22 C-Atomen, insbesondere für 2,2-Dimethyl-3-(N-morpholino)propyliden, 2,2-Dimethyl-3-lauroyloxypro-pyliden, Benzyliden oder substituiertes Benzyliden, insbesondere 4-Decylbenzyliden, 4-Undecylbenzyliden, 4-Dodecylbenzyliden, 4-Tridecylbenzyliden oder 4-Tetradecylbenzyliden, in denen die 4-Alkyl-Reste gegebenenfalls verzweigt vorliegen.

Besonders bevorzugt enthält die feuchtigkeitshärtende Polyurethan-Zusammensetzung mindestens ein Bis-Oxazolidin der Formel (VIII), bei welchem D für 1,6-Hexylen steht. Eine solche Zusammensetzung ermöglicht besonders hohe Festigkeiten bei hoher Dehnbarkeit.

Geeignete Katalysatoren sind Katalysatoren für die Beschleunigung der Reaktion von Isocyanatgruppen, insbesondere Organozinn(IV)-Verbindungen wie insbesondere Dibutylzinndiacetat, Dibutylzinndilaurat, Dibutylzinndichlorid, Dibutylzinndiacetylacetonat, Dimethylzinndilaurat, Dioctylzinndiacetat, Dioctylzinndilaurat oder Dioctylzinndiacetylacetonat, Komplexverbindungen von Bismut(III) oder Zirkonium(IV), insbesondere mit Liganden ausgewählt aus Alkoholaten, Carboxylaten, 1,3-Diketonaten, Oxinat, 1,3-Ketoesteraten und 1,3-Ketoamidaten, oder tertiäre Aminogruppen enthaltende Verbindungen wie insbesondere 2,2'-Dimorpholinodiethylether (DMDEE).

Geeignete Katalysatoren sind weiterhin Katalysatoren für die Hydrolyse von Aldimingruppen, insbesondere organische Säuren, insbesondere Carbonsäuren wie 2-Ethylhexansäure, Laurinsäure, Stearinsäure, Isostearinsäure, Ölsäure, Neodecansäure, Benzoesäure, Salicylsäure oder 2-Nitrobenzoesäure, organische Carbonsäureanhydride wie Phthalsäureanhydrid, Hexahydrophthalsäureanhydrid oder Hexahydromethylphthalsäureanhydrid, Silylester von Carbonsäuren, organische Sulfonsäuren wie Methansulfonsäure, p-Toluolsulfonsäure oder 4-Dodecylbenzolsulfonsäure, Sulfonsäureester, andere organische oder anorganische Säuren, oder Mischungen der vorgenannten Säuren und Säureester. Besonders bevorzugt sind Carbonsäuren, insbesondere aromatische Carbonsäuren wie Benzoesäure, 2-Nitrobenzoesäure oder insbesondere Salicylsäure.

Geeignet sind insbesondere auch Kombinationen von verschiedenen Katalysatoren.

Geeignete Stabilisatoren sind insbesondere Stabilisatoren gegen Oxidation, Wärme, Licht oder UV-Strahlung, insbesondere Titandioxide, Eisenoxide, Zinkoxide, Benzophenone, Benzotriazole, Verbindungen mit 2,6-Di-tert.butylphenol-Gruppen, wie sie beispielsweise unter dem Handelsnamen Irganox^{®} (von BASF) bekannt sind, Verbindungen mit 2,2,6,6-Tetramethylpiperidin-Gruppen, sogenannte HALS (hindered amine light stabilizers), wie sie beispielsweise unter dem Handelsnamen Tinuvin^{®} (von BASF) bekannt sind, oder Phosphor-haltige Verbindungen, wie sie beispielsweise unter dem Handelsnamen Irgafos^{®} (von BASF) bekannt sind.

Die feuchtigkeitshärtende Polyurethan-Zusammensetzung kann weitere Zusätze enthalten, insbesondere
- anorganische oder organische Pigmente, insbesondere Titandioxid, Chromoxide oder Eisenoxide;
- Fasern, insbesondere Glasfasern, Kohlefasern, Metallfasern, Keramikfasern, Kunststofffasern wie Polyamidfasern oder Polyethylenfasern, oder Naturfasern wie Wolle, Cellulose, Hanf oder Sisal;
- Nanofüllstoffe wie Graphen oder Carbon Nanotubes;
- Farbstoffe;
- Trocknungsmittel, insbesondere Molekularsiebpulver, Calciumoxid, hochreaktive Isocyanate wie p-Tosylisocyanat, Mono-Oxazolidine wie Incozol^{®} 2 (von Incorez) oder Orthoameisensäureester;
- Haftvermittler, insbesondere Organoalkoxysilane, insbesondere Epoxysilane wie insbesondere 3-Glycidoxypropyltrimethoxysilan oder 3-Glycidoxypropyltriethoxysilan, (Meth)acrylosilane, Anhydridosilane, Carbamatosilane, Alkylsilane oder Iminosilane, oder oligomere Formen dieser Silane, oder Titanate;
- weitere Katalysatoren, welche die Reaktion der Isocyanatgruppen beschleunigen;
- Rheologie-Modifizierer, insbesondere Verdickungsmittel, insbesondere Schichtsilikate wie Bentonite, Derivate von Rizinusöl, hydriertes Rizinusöl, Polyamide, Polyamidwachse, Polyurethane, Harnstoffverbindungen, pyrogene Kieselsäuren, Celluloseether oder hydrophob modifizierte Polyoxyethylene;
- Lösemittel, insbesondere Aceton, Methylacetat, tert.Butylacetat, 1-Methoxy-2-propylacetat, Ethyl-3-ethoxypropionat, Diisopropylether, Diethylenglykoldiethylether, Ethylenglykoldiethylether, Ethylenglykolmonobutylether, Ethylen-glykolmono-2-ethylhexylether, Acetale wie Propylal, Butylal, 2-Ethylhexylal, Dioxolan, Glycerolformal oder 2,5,7,10-Tetraoxaundecan (TOU), Toluol, Xylol, Heptan, Octan, Naphtha, White Spirit, Petrolether oder Benzin, insbesondere Solvesso^{™}-Typen (von Exxon), sowie Propylencarbonat, Dimethylcarbonat, Butyrolacton, N-Methylpyrrolidon, N-Ethylpyrrolidon, p-Chlorobenzotrifluorid oder Benzotrifluorid;
- natürliche Harze, Fette oder Öle wie Kolophonium, Schellack, Leinöl, Rizinusöl oder Sojaöl;
- nicht-reaktive Polymere, insbesondere Homo- oder Copolymere von ungesättigten Monomeren, insbesondere aus der Gruppe umfassend Ethylen, Propylen, Butylen, Isobutylen, Isopren, Vinylacetat oder Alkyl(meth)acrylate, insbesondere Polyethylene (PE), Polypropylene (PP), Polyisobutylene, Ethylenvinylacetat-Copolymere (EVA) oder ataktische Poly-α-Olefine (APAO);
- flammhemmende Substanzen, insbesondere die bereits genannten Füllstoffe Aluminiumhydroxid oder Magnesiumhydroxid, sowie insbesondere organische Phosphorsäureester wie insbesondere Triethylphosphat, Trikresylphosphat, Triphenylphosphat, Diphenylkresylphosphat, Isodecyldiphenylphosphat, Tris-(1,3-dichlor-2-propyl)phosphat, Tris(2-chlorethyl)phosphat, Tris(2-ethylhexyl)-phosphat, Tris(chlorisopropyl)phosphat, Tris(chlorpropyl)phosphat, isopropyliertes Triphenylphosphat, Mono-, Bis- oder Tris(isopropylphenyl)phosphate unterschiedlichen Isopropylierungsgrades, Resorcinol-bis(diphenylphosphat), Bisphenol-A-bis(diphenylphosphat) oder Ammoniumpolyphosphate;
- Additive, insbesondere Netzmittel, Verlaufmittel, Entschäumer, Entlüfter oder Biozide;
oder weitere üblicherweise in feuchtigkeitshärtenden Polyurethan-Zusammensetzungen eingesetzte Substanzen.

Es kann sinnvoll sein, gewisse Substanzen vor dem Einmischen in die Zusammensetzung chemisch oder physikalisch zu trocknen.

Die feuchtigkeitshärtende Polyurethan-Zusammensetzung wird insbesondere unter Ausschluss von Feuchtigkeit hergestellt und bei Umgebungstemperatur in feuchtigkeitsdichten Gebinden aufbewahrt. Ein geeignetes feuchtigkeitsdichtes Gebinde besteht insbesondere aus einem gegebenenfalls beschichteten Metall und/oder Kunststoff und ist insbesondere ein Fass, ein Container, ein Hobbock, ein Eimer, ein Kanister, eine Büchse, ein Beutel, ein Schlauchbeutel, eine Kartusche oder eine Tube.

Die feuchtigkeitshärtende Polyurethan-Zusammensetzung kann in Form einer einkomponentigen oder in Form einer mehrkomponentigen, insbesondere zweikomponentigen, Zusammensetzung vorliegen.

Als "einkomponentig" wird eine Zusammensetzung bezeichnet, bei welcher alle Bestandteile der Zusammensetzung im gleichen Gebinde vorliegen und welche als solche lagerstabil ist.

Als "zweikomponentig" wird eine Zusammensetzung bezeichnet, bei welcher die Bestandteile der Zusammensetzung in zwei verschiedenen Komponenten vorliegen, welche in voneinander getrennten Gebinden gelagert und erst kurz vor oder während der Applikation der Zusammensetzung miteinander vermischt werden.

Die feuchtigkeitshärtende Polyurethan-Zusammensetzung ist bevorzugt einkomponentig. Sie ist bei geeigneter Verpackung und Aufbewahrung lagerstabil, typischerweise während mehreren Monaten bis zu einem Jahr oder länger.

Bei der Applikation der feuchtigkeitshärtenden Polyurethan-Zusammensetzung beginnt der Prozess der Aushärtung. Als Ergebnis davon entsteht die ausgehärtete Zusammensetzung.

Im Fall einer einkomponentigen Zusammensetzung wird diese als solche appliziert und beginnt darauf unter dem Einfluss von Feuchtigkeit bzw. Wasser auszuhärten. Zur Beschleunigung der Aushärtung kann der Zusammensetzung bei der Applikation eine Beschleuniger-Komponente, welche Wasser und gegebenenfalls einen Katalysator und/oder einen Härter enthält, zugemischt werden, oder die Zusammensetzung kann nach ihrer Applikation mit einer solchen Beschleuniger-Komponente in Kontakt gebracht werden.

Bei der Aushärtung reagieren die Isocyanatgruppen unter dem Einfluss von Feuchtigkeit mit den hydrolysierenden Aldimingruppen und gegebenenfalls vorhandenen weiteren blockierten Aminogruppen und parallel dazu oder anschliessend auch untereinander zu Harnstoffgruppen. Die Gesamtheit dieser und gegebenenfalls weiterer zur Aushärtung der Zusammensetzung führenden Reaktionen der Isocyanatgruppen wird auch als Vernetzung bezeichnet.

Die zur Aushärtung der feuchtigkeitshärtenden Polyurethan-Zusammensetzung benötigte Feuchtigkeit gelangt bevorzugt aus der Luft (Luftfeuchtigkeit) durch Diffusion in die Zusammensetzung. Dabei bildet sich an den mit Luft in Kontakt stehenden Oberflächen der Zusammensetzung eine feste Schicht ausgehärteter Zusammensetzung (Haut). Die Aushärtung setzt sich entlang der Diffusionsrichtung von aussen nach innen fort, wobei die Haut zunehmend dicker wird und schliesslich die ganze applizierte Zusammensetzung umfasst. Die Feuchtigkeit kann zusätzlich oder vollständig auch aus einem oder mehreren Substrat(en), auf welche(s) die Zusammensetzung appliziert wurde, in die Zusammensetzung gelangen und/oder aus einer Beschleuniger-Komponente stammen, welche der Zusammensetzung bei der Applikation zugemischt oder nach der Applikation mit dieser in Kontakt gebracht wird, beispielsweise durch Bestreichen oder Besprühen.

Die feuchtigkeitshärtende Polyurethan-Zusammensetzung wird bevorzugt bei Umgebungstemperatur appliziert, insbesondere im Bereich von etwa -10 bis 50°C, bevorzugt im Bereich von -5 bis 45°C, insbesondere 0 bis 40°C.

Die Aushärtung der feuchtigkeitshärtenden Polyurethan-Zusammensetzung erfolgt bevorzugt bei Umgebungstemperatur.

Bevorzugt wird die feuchtigkeitshärtende Polyurethan-Zusammensetzung verwendet als Klebstoff oder Dichtstoff oder Beschichtung, insbesondere in der Bau-und Fertigungsindustrie oder im Fahrzeugbau.

Bevorzugt ist die Verwendung als elastischer Klebstoff und/oder Dichtstoff, insbesondere für die Parkettverklebung, Montage, Anbauteilverklebung, Modulverklebung, Scheibenverklebung, Fugenabdichtung, Karosserieabdichtung, Nahtabdichtung oder Hohlraumversiegelung oder für elastische Verklebungen im Fahrzeugbau wie insbesondere das Ankleben von Teilen wie Kunststoffabdeckungen, Zierleisten, Flansche, Stosstangen, Führerkabinen oder andere Anbauteile an die lackierte Karosserie eines Fahrzeugs, oder das Einkleben von Scheiben in die Karosserie, wobei die Fahrzeuge insbesondere Automobile, Lastkraftwagen, Busse, Schienenfahrzeuge oder Schiffe darstellen.

Weiterhin bevorzugt ist die Verwendung als elastische Beschichtung zum Schutz von Böden oder Mauern, insbesondere als sogenannte Flüssigfolie (liquid applied membrane) zur Abdichtung von Dächern, insbesondere Flachdächern oder schwach geneigten Dachflächen oder Dachgärten, oder im Innern von Gebäuden zur Wasserabdichtung, beispielsweise unter Fliesen oder Keramikplatten in Nasszellen oder Küchen oder auf Balkonen, oder als Nahtabdichtung, oder zu Reparaturzwecken als Abdichtung oder Beschichtung, beispielsweise von undichten Dachmembranen oder sonstigen elastischen Abdichtungen.

### Beispiele

Im Folgenden sind Ausführungsbeispiele aufgeführt, welche die beschriebene Erfindung näher erläutern sollen. Selbstverständlich ist die Erfindung nicht auf diese beschriebenen Ausführungsbeispiele beschränkt.

Als "Normklima" (NK) wird eine Temperatur von 23±1 °C und eine relative Luftfeuchtigkeit von 50±5% bezeichnet.

Die verwendeten Chemikalien waren, sofern nicht anders bezeichnet, von Sigma-Aldrich Chemie GmbH.

### Beschreibung der Messmethoden:

**Gaschromatogramme** (GC) wurden gemessen im Temperaturbereich von 60 bis 320 °C mit einer Aufheizrate von 15°C/min und 10 min Verweilzeit bei 320°C. Die Injektortemperatur betrug 250°C. Es wurde eine Zebron ZB-5 Säule verwendet (L = 30 m, ID = 0.25 mm, dj = 0.5 µm) bei einem Gasfluss von 1.5 ml/min. Die Detektion erfolgte mittels Flammenionisation (FID). Zur Zuordnung der GC-Peaks zu chemischen Strukturen wurde zudem ein Massenspektrum (EI⁺) aufgenommen.

**Infrarotspektren** (FT-IR) wurden als unverdünnte Filme auf einem FT-IR Gerät Bruker Alpha Eco-ATR gemessen. Die Absorptionsbanden sind in Wellenzahlen (cm⁻¹) angegeben.

Die **DSC** Analysen (differential scanning calorimetry) wurden mit einem Mettler Toledo DSC 3+ 700 Gerät in einem Temperaturbereich von 10 bis 400°C mit einer Aufheizrate von 4 K/min unter Verwendung von adiabatischen Drucktiegeln M20 (von TÜV Süd, Schweiz) bestimmt (first run).

Die **Aminzahl** (inklusive blockierte Aminogruppen) wurde bestimmt mittels Titration (mit 0.1_{N} HClO₄ in Essigsäure gegen Kristallviolett).

### Herstellung von Aldolestern der Formel (I):

### Beispiel 1:

### Herstellung von erfindungsgemässem Reaktionsprodukt enthaltend 2,2-Dimethyl-3-acetoxypropanal in Anwesenheit von Triethylamin

### Stufe 1 (Aldolreaktion):

In einem unter Stickstoffatmosphäre gestellten Reaktor aus V4A-Stahl, ausgerüstet mit Dosier-, Rühr-, Heiz- und Kühlsystem und einer Destillationskolonne mit Kondensator, wurden 297 kg Triethylamin (von BASF), 587 kg Paraformaldehyd (von Tennants Fine Chemicals) und 282 kg deionisiertes Wasser vorgelegt und vermischt. Die Mischung wurde unter Rühren und Rückfluss auf 60°C aufgeheizt. Dann wurden innerhalb von 3 Stunden 1523 kg Isobutyraldehyd (von BASF) zudosiert und die Reaktionsmischung dabei bei 65 bis 75°C unter Rückfluss gehalten. Nach weiteren 30 min Rückfluss war keine Exothermie mehr feststellbar. Anschliessend wurde auf Destillieren umgestellt, der Innendruck langsam reduziert und zuerst bei 85°C/250 mbar und dann bei 100°C/50 mbar die leichtflüchtigen Anteile abdestilliert. Es wurden 705 kg Destillat aufgefangen (gemäss Gaschromatographie unreagiertes Isobutyraldehyd, Wasser und ein Grossteil des Triethylamins). Im Reaktor verblieben 1924 kg Reaktionsmischung, welche gemäss Gaschromatographie ca. 88 Gewichts-% 2,2-Dimethyl-3-hydroxypropanal (Retentionszeit 3.2 min) und ca. 4 Gewichts-% Triethylamin (Retentionszeit 2.2 min) enthielt.

### Stufe 2 (Veresterung):

Anschliessend wurde der Reaktor mittels Stickstoff auf Normaldruck gebracht, auf Rückfluss eingestellt und die Innentemperatur auf 110 °C erhöht. Dann wurde der Innendruck auf 250 mbar reduziert und innerhalb von 1 Stunde 2076 kg Essigsäureanhydrid (von BP Chemicals) zugegeben und eingemischt. Anschliessend wurden die leichtflüchtigen Anteile aus der Reaktionsmischung entfernt. Dazu wurde der Reaktor auf fraktionierte Destillation eingestellt (80% Rückfluss) und bei einer Kopftemperatur von ca. 78°C destilliert. Sobald die Kopftemperatur 80°C erreichte, wurde der Innendruck des Reaktors langsam weiter reduziert und jeweils solange destilliert, bis die Kopftemperatur wieder 80°C erreichte. Als bei einem Innendruck von 30 mbar die Kopftemperatur 80°C überschritt, wurde die Destillation, bzw. das Entfernen der leichtflüchtigen Anteile aus der Reaktionsmischung, beendet. Es wurden insgesamt 2134 kg Destillat aufgefangen (gemäss Gaschromatographie unreagiertes Essigsäureanhydrid, Essigsäure, Triethylamin und 2,2-Dimethyl-3-acetoxypropanal). Dann wurde das Reaktionsprodukt abgekühlt und unter Stickstoffatmosphäre gestellt.

Erhalten wurden 1851 kg einer klaren, hellfarbig gelblichen, mild fruchtig riechenden Flüssigkeit. Das Reaktionsprodukt enthielt gemäss Gaschromatographie ca. 78 Gewichts-% 2,2-Dimethyl-3-acetoxypropanal (Retentionszeit 4.8 min), ca. 5.7 Gewichts-% Triester der Formel (V) (Retentionszeit 10.9 min) und ca. 6.3 % Gewichts-% Acetal der Formel (VI) (Retentionszeit 6.4 min und 6.6 min). Es wird im Folgenden als "Reaktionsprodukt aus Beispiel 1" bezeichnet.

FT-IR: 2973, 2938, 2877, 2818, 2716, 1728, 1473, 1374, 1228, 1160, 1118, 1040, 892, 775.

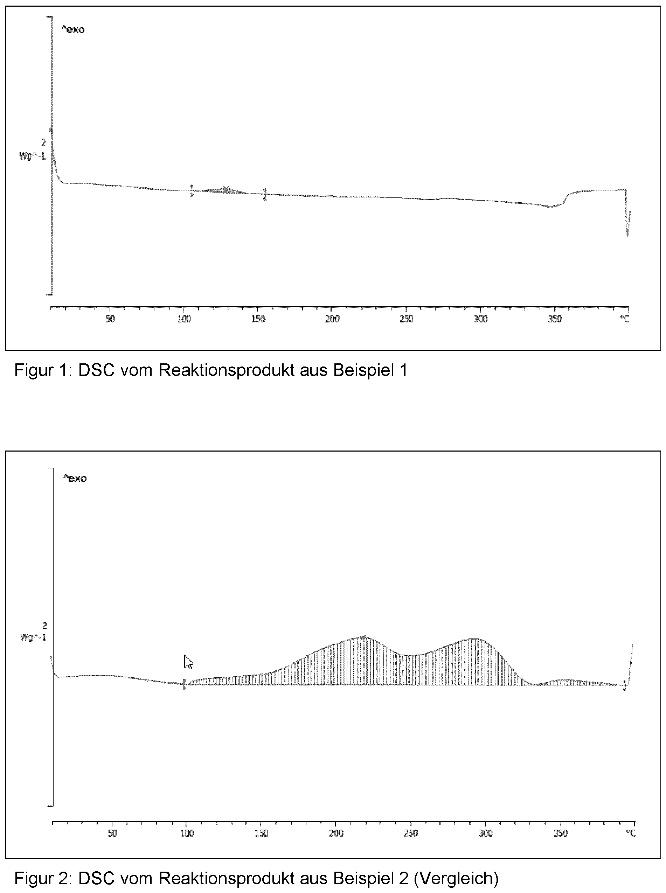

Vom Reaktionsprodukt wurde ein DSC aufgenommen, welches in Figur 1 dargestellt ist. Dabei wurde eine schwache Exothermie im Bereich von 105 bis 155°C von 20 kJ/kg bestimmt.

### Reinigung des Reaktionsprodukts mittels über Kopf Destillation: (als Vergleich)

500 g des erhaltenen Reaktionsprodukts aus Beispiel 1 wurden bei 120 bis 130°C in einem Rundkolben mit Destillationskolonne unter Vakuum destilliert. Dabei wurden 370.4 g Destillat (= über Kopf destilliertes 2,2-Dimethyl-3-acetoxypropanal aus Beispiel 1) bei einer Kopftemperatur von 84 bis 87°C, 30 mbar und 60% Rückfluss erhalten, welches gemäss Gaschromatographie ca. 94 Gewichts-% 2,2-Dimethyl-3-acetoxypropanal enthielt.

Die erste Fraktion (= Vorlauf) von 73.8 g wurde bei einer Kopftemperatur von 76 bis 80°C, 30 mbar und 80% Rückfluss aufgefangen. Sie enthielt gemäss Gaschromatographie ca. 56 Gewichts-% 2,2-Dimethyl-3-acetoxypropanal, ca. 17 Gewichts-% Essigsäure und ca. 18 Gewichts-% Triethylamin. Als Rückstand verblieben 55.8 g mit einem Gehalt an 2,2-Dimethyl-3-acetoxypropanal von 0.8 Gewichts-%.

### Beispiel 2: (Vergleich)

### Herstellung von 2,2-Dimethyl-3-acetoxypropanal in Anwesenheit von Säure

In einem Rundkolben mit Destillationskolonne und Wasserabscheider wurden unter Stickstoffatomosphäre 100 g Cyclohexan, 144.0 g Parafomaldehyd, 403.7 g Essigsäure und 6.3 g para-Toluolsulfonsäure vorgelegt und vermischt. Die Mischung wurde unter gutem Rühren und Rückfluss auf 60°C aufgeheizt und langsam 346.4 g Isobutyraldehyd zugegeben, so dass die Innentemperatur nicht über 75°C stieg. Anschliessend wurde von Rückfluss auf Wasserabscheiden umgestellt und langsam auf 100°C Innentemperatur aufgeheizt. Als die Innentemperatur 100°C erreicht hatte, wurde der Innendruck langsam reduziert, wobei darauf geachtet wurde, dass die Innentemperatur ungefähr bei 100°C gehalten wurde. Bei einem Innendruck von 600 mbar waren 81 g Wasser abgeschieden. Dann wurde von Wasserabscheiden auf Destillieren umgestellt und der Innendruck weiter reduziert, so dass die Innentemperatur ungefähr bei 100°C gehalten wurde. Bei einem Innendruck von 30 mbar und einer Kopftemperatur von 67°C war die überschüssige Essigsäure mehrheitlich entfernt. Das Reaktionsprodukt wurde abgekühlt und unter Stickstoffatmosphäre gestellt. Das aufgefangene Destillat bestand gemäss Gaschromatographie mehrheitlich aus Cyclohexan, etwas Wasser, Isobutyraldehyd und Essigsäure.

Erhalten wurden 576 g einer dunkel gefärbten, stechend riechenden Flüssigkeit. Das Reaktionsprodukt enthielt gemäss Gaschromatographie ca. 61.7 Gewichts-% 2,2-Dimethyl-3-acetoxypropanal (Retentionszeit 4.8 min).

Vom Reaktionsprodukt aus Beispiel 2 wurde ein DSC aufgenommen, welches in Figur 2 dargestellt ist. Dabei wurde eine starke Exothermie im Bereich von 100 bis 400°C von 530 kJ/kg bestimmt.

### Herstellung von blockierten Aminen:

### Aldimin A1: (aus erfindungsgemässem Reaktionsprodukt)

### N,N'-Bis(2,2-dimethyl-3-acetoxypropyliden)-3-aminomethyl-3,5,5-trimethylcyclohexylamin

373.0 g des Reaktionsprodukts aus Beispiel 1 enthaltend ca. 78 Gewichts-% 2,2-Dimethyl-3-acetoxypropanal wurden unter Stickstoffatmosphäre in einem Rundkolben vorgelegt. Dann wurden 170.3 g (1 mol) 3-Aminomethyl-3,5,5-trimethylcyclohexylamin (Vestamin^{®} IPD, von Evonik) unter gutem Rühren zugegeben und anschliessend die flüchtigen Bestandteile bei 80°C und 10 mbar Vakuum entfernt. Erhalten wurden 497 g einer klaren, hellfarbig gelblichen, niedrigviskosen, mild fruchtig riechenden Flüssigkeit mit einer Aminzahl von 223 mg KOH/g, was einem berechneten Aldimin-Equivalentgewicht von 252 g/eq entspricht.

### Aldimin R1: (Vergleich, aus gereinigtem Reaktionsprodukt)

### N,N'-Bis(2,2-dimethyl-3-acetoxypropyliden)-3-aminomethyl-3,5,5-trimethylcyclohexylamin

293 g über Kopf destilliertes 2,2-Dimethyl-3-acetoxypropanal aus Beispiel 1 wurden unter Stickstoffatmosphäre in einem Rundkolben vorgelegt. Dann wurden 170.3 g (1 mol) 3-Aminomethyl-3,5,5-trimethylcyclohexylamin (Vestamin^{®} IPD, von Evonik) unter gutem Rühren zugegeben und anschliessend die flüchtigen Bestandteile bei 80°C und 10 mbar Vakuum entfernt. Erhalten wurden 418 g einer klaren, fast farblosen, niedrigviskosen, mild fruchtig riechenden Flüssigkeit mit einer Aminzahl von 262 mg KOH/g, was einem berechneten Aldimin-Equivalentgewicht von 214 g/eq entspricht.

### Feuchtigkeitshärtende Polyurethan-Zusammensetzungen:

### Zusammensetzungen Z1 und Z2

Für jede Zusammensetzung wurden die folgenden Inhaltsstoffe unter Ausschluss von Feuchtigkeit in einem Zentrifugalmischer vermischt, bis eine makroskopisch homogene Flüssigkeit entstanden war:
213.7 g eines Isocyanatgruppen-haltigen Polymers mit einem NCO-Gehalt von 3.7 Gewichts-%, basierend auf einem Polyoxypropylendiol mit OH-Zahl 56 mg KOH/g und Toluendiisocyanat (Desmodur^{®} T 80 P, von Covestro), 61.3 g Vernetzer (Desmodur^{®} L67 MPA/X, von Covestro), 73 g Weichmacher, 149 g Lösemittel, 19 g Verdicker, 417 g anorganische Füllstoffe und 0.5 g Salicylsäure. Zusätzlich wurden für die Zusammensetzung **Z1** 67.7 g **Aldimin A1** bzw. für die Zusammensetzung **Z2** 57.5 g **Aldimin R1** zugegeben.

Jede Zusammensetzung wurde in einem dicht verschlossenen Metallgebinde unter Ausschluss von Feuchtigkeit aufbewahrt und schliesslich folgendermassen geprüft:
Die **Viskosität** wurde bestimmt mit einem Rotothinner bei 20°C. "frisch" steht für die gemessene Viskosität 24 h nach der Herstellung der Zusammensetzung. "4w 40°C" bzw. "8w 40°C" steht für die Viskosität nach einer Lagerung während 4 Wochen bzw. 8 Wochen bei 40°C im verschlossenen Gebinde.

Die Aushärtungsgeschwindigkeit **("BK Trockenzeit")** wurde bestimmt im Nomklima mit einem Beck-Koller Trockenzeit Rekorder nach ASTM D5895. Das Resultat für Phase 2 entspricht der Hautbildungszeit (tack free time) der Zusammensetzung.

Die **Durchhärtung** wurde bestimmt, indem die Zusammensetzung in Form eines Zylinders von 40 mm Durchmesser und 4 mm Höhe appliziert, im Normklima (NK) oder bei 5°C/80% relativer Feuchtigkeit stehen gelassen, nach 24 h oder 48 h aufgeschnitten und die Dicke der ausgehärteten Schicht, die sich auf der Oberfläche der Zusammensetzung gebildet hatte, gemessen wurde. Die Resultate sind angegeben als "24h NK" bzw. "48h NK" bzw. "48h 5°C", je nach Zeitdauer und Klima bei der Aushärtung.

Zur Bestimmung der mechanischen Eigenschaften wurde für jede Zusammensetzung ein zweischichtiger ausgehärteter Film hergestellt. Dazu wurde eine erste Schicht von 800 µm Dicke mit einem Rakel appliziert und während 24 h im Normklima gelagert, gefolgt von einer zweiten Schicht, welche in einer Dicke von 400 µm in einem Winkel von 90° relativ zur ersten Schicht mit einem Rakel appliziert wurde. Dieser zweischichtige Film wurde weitere 24 h im Normklima gelagert, gefolgt von 24 h in einem Umluftofen bei 60 °C. Nach weiteren 24 h in Nomklima wurden streifenförmige Prüfkörper von 100 mm Länge und 25 mm Breite aus dem Film ausgestanzt und damit die **Zugfestigkeit** und die **Bruchdehnung** in Anlehnung an DIN EN 53504 bei einer Zuggeschwindigkeit von 180 mm/min bei einer Spurlänge von 60 mm bestimmt.

Der **Aspekt** wurde optisch am Film, welcher für die Bestimmung der mechanischen Eigenschaften hergestellt wurde, bestimmt.

Der **Geruch** wurde durch Riechen mit der Nase in einem Abstand von etwa 100 mm an einer frisch applizierten, flächigen Zusammensetzung von etwa 150 mm Durchmesser bestimmt.

**Tabelle 1: Eigenschaften der Zusammensetzungen Z1 und Z2.**

| **Zusammensetzung** | | | **Z1** | **Z2 (Vergleich)** |
|---|---|---|---|---|
| **Viskosität** [mPa·s] | | frisch | 1'800 | 1'950 |
| | 4 Wochen 40°C | | 2'200 | 2'400 |
| | 8 Wochen 40°C | | 2'350 | 2'500 |
| **BK Trockenzeit** | | Phase 2 | 1:38 | 1:30 |
| [h:min] | | Phase 4 | 2:53 | 3:00 |
| **Durchhärtung** | | 24h NK | 2.6 | 2.6 |
| (mm Tiefe) | | 48h NK | 3.9 | 4.0 |
| | | 48h 5°C | 3.8 | 3.8 |
| **Zugfestigkeit** [MPa] | | | 5.59 | 5.50 |
| **Bruchdehnung** [%] | | | 328 | 260 |
| **Aspekt** | | | matt, nicht-klebrig, keine Blasen | matt, nicht-klebrig, keine Blasen |
| **Geruch** | | | mild, lösemittelartig, leicht fruchtig | mild, lösemittelartig, leicht fruchtig |

Aus der Tabelle 1 ist ersichtlich, dass das erfindungsgemässe Reaktionsprodukt aus Beispiel 1 als solches, also ohne weitere Aufreinigung mittels Destillation über Kopf, hervorragend geeignet ist für die Herstellung des Aldimins **A1**, welches als blockiertes Amin bwz. latenter Härter in einer einkomponentigen feuchtigkeitshärtenden Zusammensetzung eingesetzt wird. Überraschenderweise zeigt die Zusammensetzung **Z1** teilweise sogar bessere Eigenschaften im Vergleich zur Zusammensetzung **Z2,** welche das Aldimin **R1** abgeleitet von mittels Destillation über Kopf gereinigtem 2,2-Dimethyl-3-acetoxypropanal enthält. Insbesondere zeigt die Zusammensetzung **Z1** eine besonders niedrigere Viskosität, auch nach Lagerung, und eine besonders hohe Dehnung, bei sonst vergleichbaren Eigenschaften.

Die Zusammensetzungen **Z1** und **Z2** sind insbesondere geeignet als Beschichtung oder Belag, insbesondere als sogenannte Flüssigfolie (liquid applied membrane) für die Abdichtung von Dächern, Brücken, Terassen usw.

## Patentansprüche

1. Verfahren zur Herstellung eines Aldolesters der Formel (I), 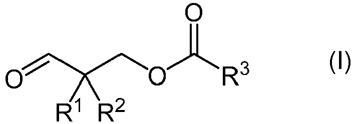 wobei R¹ und R² für gleiche oder verschiedene Alkyl-Reste mit 1 bis 4 C-Atomen oder zusammen für einen Alkylen-Rest mit 4 bis 6 C-Atomen stehen und R³ für einen gegebenenfalls halogenierten Kohlenwasserstoff-Rest mit 1 bis 17 C-Atomen steht,
**dadurch gekennzeichnet, dass** mindestens ein Carbonsäureanhydrid der Formel (II) 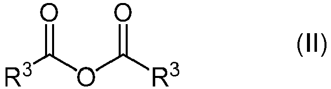 mit mindestens einem Aldol der Formel (III), 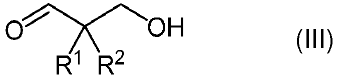 gegebenenfalls in Form eines Oligomers davon, unter Erwärmen in Gegenwart eines basischen Katalysators mit einem pKa-Wert der konjugierten Säure von mindestens 8 umgesetzt wird, wobei das Aldol der Formel (III) als Bestandteil einer Reaktionsmischung eingesetzt wird, welche erhalten wurde aus der Umsetzung von Formaldehyd, gegebenenfalls in Form von Paraformaldehyd oder Trioxan, mit mindestens einem Aldehyd der Formel (IV) 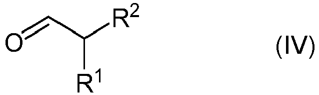 in Gegenwart eines basischen Katalysators mit einem pKa-Wert der konjugierten Säure von mindestens 8, und wobei die Reaktionsmischung enthaltend das Aldol der Formel (III) frei von starken Säuren ist, insbesondere halogenhaltigen Säuren wie Bortrichlorid, Bortribromid oder Salzsäure.

2. Verfahren gemäss Anspruch 1, **dadurch gekennzeichnet, dass** R¹ und R² jeweils für Methyl stehen.

3. Verfahren gemäss einem der Ansprüche 1 bis 2, **dadurch gekennzeichnet, dass** R³ für einen Alkyl-Rest mit 1 bis 7 C-Atomen oder für Phenyl steht, insbesondere für Methyl.

4. Verfahren gemäss einem der Ansprüche 1 bis 3, **dadurch gekennzeichnet, dass** der basische Katalysator einen pKa-Wert der konjugierten Säure von mindestens 9, insbesondere mindestens 10, aufweist.

5. Verfahren gemäss einem der Ansprüche 1 bis 4, **dadurch gekennzeichnet, dass** der basische Katalysator Triethylamin ist.

6. Verfahren gemäss einem der Ansprüche 1 bis 5, **dadurch gekennzeichnet, dass** es bei einer Temperatur im Bereich von 80 bis 150°C, insbesondere 100 bis 130°C, durchgeführt wird.

7. Verfahren gemäss einem der Ansprüche 1 bis 6, **dadurch gekennzeichnet, dass** es ohne die Verwendung eines organischen Löse- oder Schleppmittels durchgeführt wird.

8. Verfahren gemäss einem der Ansprüche 1 bis 7, **dadurch gekennzeichnet, dass** es in zwei Stufen durchgeführt wird, wobei
(i) in der ersten Stufe der basische Katalysator und Formaldehyd, insbesondere in Form von Paraformaldehyd, vorgelegt werden, dann mindestens ein Aldehyd der Formel (IV) 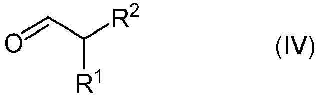 im stöchiometrischen Überschuss in Bezug auf Formaldehyd bei einer Temperatur im Bereich von 60 bis 90 °C unter Bildung des Aldols der Formel (III) zugegeben wird und anschliessend leichtsiedende Anteile aus der Reaktionsmischung entfernt werden, und
(ii) in der zweiten Stufe die so erhaltene Reaktionsmischung mit dem Carbonsäureanhydrid der Formel (II) bei einer Temperatur im Bereich von 100 bis 130°C umgesetzt wird, wobei während und/oder nach der Umsetzung leichtsiedende Anteile aus der Reaktionsmischung entfernt werden.

9. Reaktionsprodukt erhalten aus dem Verfahren gemäss einem der Ansprüche 1 bis 8, **dadurch gekennzeichnet, dass** 60 bis 95 Gewichts-% Aldolester der Formel (I) und 5 bis 40 Gewichts-% andere, nicht der Formel (I) entsprechende Ester, Aldehyde und/oder Acetale enthalten sind.

10. Reaktionsprodukt gemäss Anspruch 9, **dadurch gekennzeichnet, dass** Triester der Formel (V) und/oder Acetale der Formel (VI) enthalten sind, 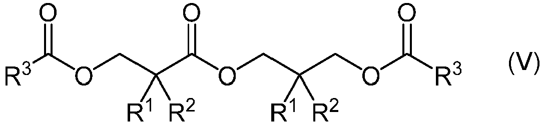 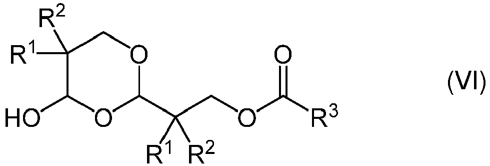 insbesondere 0.1 bis 20 Gewichts-% Triester der Formel (V) und 1 bis 20 Gewichts-% Acetale der Formel (VI).

11. Blockiertes Amin, erhalten aus der Umsetzung des Reaktionsprodukts gemäss einem der Ansprüche 9 bis 10 mit mindestens einem Amin, welches eine primäre Aminogruppe und zusätzlich mindestens eine Reaktivgruppe ausgewählt aus primärer Aminogruppe, sekundärer Aminogruppe und Hydroxylgruppe aufweist.

12. Blockiertes Amin gemäss Anspruch 11, **dadurch gekennzeichnet, dass** das Amin ausgewählt ist aus der Gruppe bestehend aus 1,6-Hexandiamin, 1-Amino-3-aminomethyl-3,5,5-trimethylcyclohexan, 4(2)-Methyl-1,3-cyclohexandiamin, 1,3-Bis(aminomethyl)cyclohexan, 1,4-Bis(aminomethyl)cyclohexan, 1,3-Bis(aminomethyl)benzol, 1,2-Cyclohexandiamin, 1,3-Cyclohexandiamin, 1,4-Cyclohexandiamin, Bis(4-aminocyclohexyl)methan, 2,5(2,6)-Bis-(aminomethyl)bicyclo[2.2.1]heptan, 3(4),8(9)-Bis(aminomethyl)tricyclo-[5.2.1.0^{2,6}]decan, α,ω-Polyoxypropylendiamin mit einem mittleren Molekulargewicht Mₙ im Bereich von 170 bis 500 g/mol, Trimethylolpropan- oder Glycerin-gestartetes Tris(ω-polyoxypropylenamin) mit einem mittleren Molekulargewicht Mₙ im Bereich von 330 bis 500 g/mol, 1,4-Phenylendiamin, 3,5-Diethyl-2,4(6)-toluylendiamin, 2-(2-Aminoethoxy)ethanol, 2-(2-(2-Amino-ethoxy)ethoxy)ethanol und 3-Aminomethyl-3,5,5-trimethylcyclohexanol.

13. Polyurethan-Zusammensetzung enthaltend
- mindestens ein Polyisocyanat und/oder Isocyanatgruppen-haltiges Polymer und
- mindestens ein blockiertes Amin gemäss einem der Ansprüche 11 bis 12.

14. Verwendung der Polyurethan-Zusammensetzung gemäss Anspruch 13 als Klebstoff oder Dichtstoff oder Beschichtung.

## Claims

1. Method for preparing an aldol ester of the formula (I) , 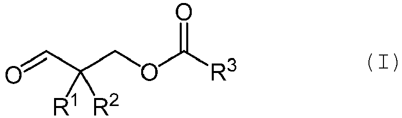 where R¹ and R² are identical or different alkyl radicals having 1 to 4 carbon atoms or together are an alkylene radical having 4 to 6 carbon atoms, and R³ is an optionally halogenated hydrocarbyl radical having 1 to 17 carbon atoms,
**characterized in that** at least one carboxylic anhydride of the formula (II) 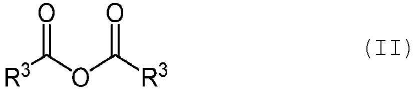 is reacted with at least one aldol of the formula (III) , 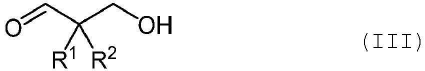 optionally in the form of an oligomer thereof, while heating in the presence of a basic catalyst having a conjugate acid pKa of at least 8, wherein the aldol of the formula (III) is used as constituent of a reaction mixture obtained from the reaction of formaldehyde, optionally in the form of paraformaldehyde or trioxane, with at least one aldehyde of the formula (IV) 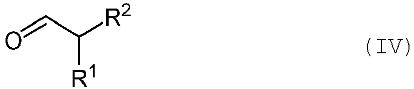 in the presence of a basic catalyst having a conjugate acid pKa of at least 8, and wherein the reaction mixture containing the aldol of the formula (III) is free of strong acids, in particular halogen-containing acids such as boron trichloride, boron tribromide or hydrochloric acid.

2. Method according to Claim 1, **characterized in that** R¹ and R² are each methyl.

3. Method according to either of Claims 1 and 2, **characterized in that** R³ is an alkyl radical having 1 to 7 carbon atoms or is phenyl, in particular is methyl.

4. Method according to any of Claims 1 to 3, **characterized in that** the basic catalyst has a conjugate acid pKa of at least 9, in particular at least 10.

5. Method according to any of Claims 1 to 4, **characterized in that** the basic catalyst is triethylamine.

6. Method according to any of Claims 1 to 5, **characterized in that** it is executed at a temperature within a range from 80 to 150°C, in particular 100 to 130°C.

7. Method according to any of Claims 1 to 6, **characterized in that** it is executed without using an organic solvent or entraining agent.

8. Method according to any of Claims 1 to 7, **characterized in that** it is executed in two steps, wherein
(i) in the first step, the basic catalyst and formaldehyde, in particular in the form of paraformaldehyde, are initially charged, then at least one aldehyde of the formula (IV) 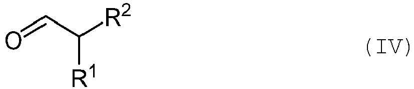 is added in stoichiometric excess in relation to formaldehyde at a temperature in the range from 60 to 90°C, resulting in the formation of the aldol of the formula (III), after which volatiles are removed from the reaction mixture, and
(ii) in the second step, the reaction mixture thus obtained is reacted with the carboxylic anhydride of the formula (II) at a temperature in the range from 100 to 130°C, wherein volatiles are removed from the reaction mixture during and/or after the reaction.

9. Reaction product obtained from the method according to any of Claims 1 to 8, **characterized in that** it comprises 60% to 95% by weight of aldol ester of the formula (I) and 5% to 40% by weight of other esters, aldehydes and/or acetals not corresponding to the formula (I) .

10. Reaction product according to Claim 9, **characterized in that** it comprises triesters of the formula (V) and/or acetals of the formula (VI), 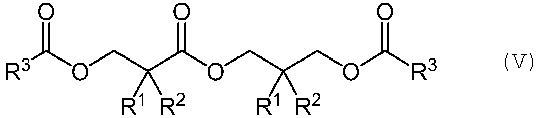 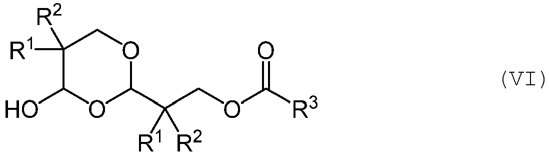 in particular 0.1% to 20% by weight of triesters of the formula (V) and 1% to 20% by weight of acetals of the formula (VI).

11. Blocked amine obtained from reacting the reaction product according to either of Claims 9 and 10 with at least one amine that has a primary amino group and additionally at least one reactive group selected from primary amino group, secondary amino group, and hydroxyl group.

12. Blocked amine according to Claim 11, **characterized in that** the amine is selected from the group consisting of hexane-1,6-diamine, 1-amino-3-aminomethyl-3,5,5-trimethylcyclohexane, 4(2)-methylcyclohexane-1,3-diamine, 1,3-bis(aminomethyl)cyclohexane, 1,4-bis(aminomethyl)cyclohexane, 1,3-bis(aminomethyl)benzene, cyclohexane-1,2-diamine, cyclohexane-1,3-diamine, cyclohexane-1,4-diamine, bis(4-aminocyclohexyl)methane, 2,5(2,6)-bis(aminomethyl)bicyclo[2.2.1]heptane, 3(4),8(9)-bis(aminomethyl)tricyclo[5.2.1.0^{2,6}]decane, α,ω-polyoxypropylenediamine having an average molecular weight Mₙ in the range from 170 to 500 g/mol, trimethylolpropane- or glycerol-started tris(ω-polyoxypropyleneamine) having an average molecular weight Mₙ in the range from 330 to 500 g/mol, 1,4-phenylenediamine, 3,5-diethyl-2,4(6)-tolylenediamine, 2-(2-aminoethoxy)ethanol, 2-(2-(2-aminoethoxy)ethoxy)ethanol, and 3-aminomethyl-3,5,5-trimethylcyclohexanol.

13. Polyurethane composition comprising
- at least one polyisocyanate and/or polymer containing isocyanate groups and
- at least one blocked amine according to either of Claims 11 and 12.

14. Use of the polyurethane composition according to Claim 13 as adhesive or sealant or coating.

## Revendications

1. Procédé pour la préparation d'un ester d'aldol de formule (I), 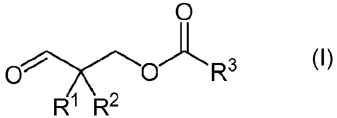 dans laquelle R¹ et R² représentent des radicaux alkyle identiques ou différents, comprenant 1 à 4 atomes de carbone ou, conjointement, un radical alkyle comprenant 4 à 6 atomes de carbone et R³ représente un radical hydrocarboné le cas échéant halogéné comprenant 1 à 17 atomes de carbone, **caractérisé en ce qu'**au moins un anhydride d'acide carboxylique de formule (II) 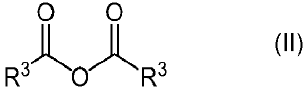 est transformé avec au moins un aldol de formule (III), 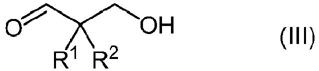 le cas échéant sous forme d'un oligomère de celui-ci, tout en chauffant en présence d'un catalyseur basique présentant une valeur pKa de l'acide conjugué d'au moins 8, l'aldol de formule (III) étant utilisé sous forme de constituant d'un mélange réactionnel qui a été obtenu à partir de la transformation de formaldéhyde, le cas échéant sous forme de paraformaldéhyde ou de trioxane, avec au moins un aldéhyde de formule (IV) 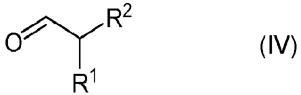 en présence d'un catalyseur basique présentant une valeur pKa de l'acide conjugué d'au moins 8 et le mélange réactionnel contenant l'aldol de formule (III) étant exempt d'acides forts, en particulier d'acides halogénés tels que le trichlorure de bore, le tribromure de bore ou l'acide chlorhydrique.

2. Procédé selon la revendication 1, **caractérisé en ce que** R¹ et R² représentent à chaque fois méthyle.

3. Procédé selon l'une des revendications 1 à 2, **caractérisé en ce que** R³ représente un radical alkyle comprenant 1 à 7 atomes de carbone ou phényle, en particulier méthyle.

4. Procédé selon l'une des revendications 1 à 3, **caractérisé en ce que** le catalyseur basique présente une valeur pKa de l'acide conjugué d'au moins 9, en particulier d'au moins 10.

5. Procédé selon l'une des revendications 1 à 4, **caractérisé en ce que** le catalyseur basique est la triéthylamine.

6. Procédé selon l'une des revendications 1 à 5, **caractérisé en ce qu'**il est réalisé à une température dans la plage de 80 à 150°C, en particulier de 100 à 130°C.

7. Procédé selon l'une des revendications 1 à 6, **caractérisé en ce qu'**il est réalisé sans utilisation d'un solvant ou d'un agent d'entraînement.

8. Procédé selon l'une des revendications 1 à 7, **caractérisé en ce qu'**il est réalisé en deux étapes, où
(i) dans la première étape, le catalyseur basique et le formaldéhyde, en particulier sous forme de paraformaldéhyde, sont disposés au préalable puis au moins un aldéhyde de formule (IV) 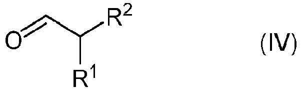 est ajouté en excès stoechiométrique par rapport au formaldéhyde à une température dans la plage de 60 à 90 °C avec formation de l'aldol de formule (III) et les proportions à bas point d'ébullition sont ensuite éliminées du mélange réactionnel et
(ii) dans la deuxième étape, le mélange réactionnel ainsi obtenu est transformé avec l'anhydride d'acide carboxylique de formule (II) à une température dans la plage de 100 à 130°C, les proportions à bas point d'ébullition étant éliminées du mélange réactionnel pendant et/ou après la transformation.

9. Produit de réaction, obtenu à partir du procédé selon l'une des revendications 1 à 8, **caractérisé en ce qu'**il contient 60 à 95% en poids d'ester d'aldol de formule (I) et 5 à 40% en poids d'autres esters, aldéhydes et/ou acétals ne correspondant pas à la formule (I).

10. Produit de réaction selon la revendication 9, **caractérisé en ce que** des triesters de formule (V) et/ou des acétals de formule (VI) sont contenus, 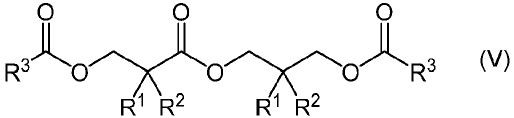 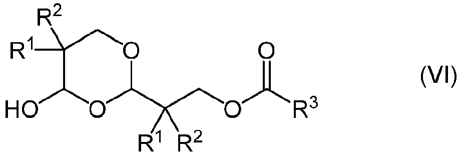 en particulier 0,1 à 20% en poids de triesters de formule (V) et 1 à 20% en poids d'acétals de formule (VI).

11. Amine bloquée, obtenue à partir de la transformation du produit de réaction selon l'une des revendications 9 à 10 avec au moins une amine, qui présente au moins un groupe amino primaire et en plus au moins un groupe réactif choisi parmi un groupe amino primaire, un groupe amino secondaire et un groupe hydroxyle.

12. Amine bloquée selon la revendication 11, **caractérisée en ce que** l'amine est choisie dans le groupe constitué par la 1,6-hexanediamine, le 1-amino-3-aminométhyl-3,5,5-triméthylcyclohexane, la 4(2)-méthyl-1,3-cyclohexanediamine, le 1,3-bis(aminométhyl)cyclohexane, le 1,4-bis(aminométhyl)cyclohexane, le 1,3-bis(aminométhyl)benzène, la 1,2-cyclohexanediamine, la 1,3-cyclohexanediamine, la 1,4-cyclohexanediamine, le bis(4-aminocyclohexyl)méthane, le 2,5(2,6)-bis-(aminométhyl)bicyclo[2.2.1]heptane, le 3(4),8(9)-bis (aminométhyl)tricyclo-[5.2.1.0^{2,6}]décane, l'α,ω-polyoxypropylènediamine présentant un poids moléculaire moyen Mₙ dans la plage de 170 à 500 g/mole, la tris (ω-polyoxypropylène-amine) démarrée par triméthylolpropane ou par glycérol présentant un poids moléculaire moyen Mₙ dans la plage de 330 à 500 g/mole, la 1,4-phénylènediamine, la 3,5-diéthyl-2,4(6)-toluylènediamine, le 2-(2-aminoéthoxy)éthanol, le 2-(2-(2-aminoéthoxy)éthoxy)éthanol et le 3-aminométhyl-3,5,5-triméthylcyclohexanol.

13. Composition de polyuréthane, contenant :
- au moins un polyisocyanate et/ou polymère contenant des groupes isocyanate et
- au moins une amine bloquée selon l'une des revendications 11 à 12.

14. Utilisation de la composition de polyuréthane selon la revendication 13 comme adhésif ou masse d'étanchéité ou revêtement.
